# EUROPEAN PATENT APPLICATION

(11) **EP 4 497 445 A1**
(43) Date of publication of application: **29.01.2025**
(21) Application number: 23774838.9
(22) Date of filing: 19.03.2023
(51) Int. Cl.: A61K 48/00, A61P 43/00, C12N 15/113, A61K 31/7105, A61K 35/32, A61P 25/28

(54) **GENE EXPRESSION REGULATOR, PROPHYLACTIC DRUG OR THERAPEUTIC DRUG FOR ALZHEIMER'S DISEASE, AND METHOD FOR IMPROVING DEMENTIA**

(30) Priority: 22.03.2022 JP 2022044972
(71) Applicant: Dexon Pharmaceuticals Inc., Tokyo, 103-0027 (JP)
(72) Inventor: KOGA Shoji, Tokyo 102-0082 (JP)
(74) Representative: Godemeyer Blum Lenze Patentanwälte Partnerschaft mbB - werkpatent
(86) International application number: PCT/JP2023/010775
(87) International publication number: WO 2023/182243

(57) **Abstract**

A gene expression regulator containing microparticles containing miRNA which targets a gene related to the expression of at least one kind of protein of amyloid precursor protein (APP), β-secretase (BACE1), an NMDA-activating protein, glycogen synthase kinase-3β (GSK-3β) and polyglutamine-binding protein-1 (PQBP1) can provide a novel gene expression regulator which can regulate (suppress, inhibit or the like) the expression of a protein related to amyloid β-related or tau protein-related dementia or brain inflammation such as Alzheimer's disease; an agent for preventing or treating Alzheimer's disease; and a method for improving dementia.

## Description

### Technical Field

The present invention relates to a gene expression regulator, an agent for preventing or treating Alzheimer's disease and a method for improving dementia.

### Background Art

Alzheimer's disease is a neurodegenerative disease which currently accounts for a half or more of the causes of dementia, and further development of therapeutic methods and preventive methods is desired. From the previous research, elucidation of the mechanism of development of Alzheimer's disease has been advanced. To prevent Alzheimer's disease, suppress the progression thereof or improve Alzheimer's disease, suppression of increase, agglutination, accumulation or deposition of amyloid β protein or tau protein, which is a microtubule-associated protein, or the like is believed to be one of important points. Agents for treating Alzheimer's disease and the mechanisms of action thereof have been found although the details will be described below (for example, see NPL 1).

Here, involvement of neuron-derived exosomes in the metabolism of brain amyloid β protein and in the development or the progression of Alzheimer's disease has been suggested (for example, see PTL 1).

PTL 1 focuses on the insufficient examination on drugs which can promote the production of exosomes and discloses an exosome production promotor containing a substance capable of inhibiting the function of and/or suppressing the expression of acid ceramidase as an active ingredient as an exosome production promotor which can easily promote the production of exosomes *in vivo.*

### Citation List

### Patent Literature

PTL 1: JP2021-083415A

### Non Patent Literature

NPL 1: Rinsho Shinkeigaku (Clinical Neurology) 54(12), 1178-1180, 2014

### Summary of Invention

### Technical Problem

As it is obvious from the method for solving the problem, however, PTL 1 focuses on human neurons, in which accumulation of amyloid β or the like actually occurs, but does not focus on exosomes derived from other cells. For example, PTL 1 merely describes an example in which the production of exosomes was promoted in human neuroblastoma-derived SH-SY5Y cells. Moreover, the documents cited in PTL 1 are a non-patent literature which suggests that neuron-derived exosomes highly express amyloid β-binding glycolipid, have the capability of capturing amyloid β protein and have the action of removing amyloid β protein in cooperation with brain phagocytic microglia, a non-patent literature which suggests that neuron-derived exosomes capture amyloid β on the surface thereof, transport to microglia and thus improve the efficiency of degradation of amyloid β protein and the like.

A problem to be solved by the invention is to provide a novel gene expression regulator which can regulate (suppress, inhibit or the like) the expression of a protein related to amyloid β-related or tau protein-related dementia or brain inflammation such as Alzheimer's disease.

### Solution to Problem

The present inventor and the like have found that microparticles containing specific microRNA can regulate (suppress, inhibit or the like) the expression of a protein related to amyloid β-related or tau protein-related dementia or brain inflammation.

Specifically, the invention and preferable configurations of the invention are as follows.
[1] A gene expression regulator containing microparticles containing miRNA which targets a gene related to the expression of at least one kind of protein of amyloid precursor protein (APP), β-secretase (BACE1), an NMDA-activating protein, glycogen synthase kinase-3β (GSK-3β) and polyglutamine-binding protein-1 (PQBP1).
[2] The gene expression regulator according to [1], in which the microparticles are exosomes.
[3] The gene expression regulator according to [1] or [2], in which the microparticles contain the miRNA which targets the gene related to the expression of at least one kind of protein of APP, BACE1, the NMDA-activating protein, GSK-3β and PQBP1 at a higher concentration than that of a culture supernatant of dental pulp-derived stem cells.
[4] The gene expression regulator according to any one of [1] to [3]
   which is an expression suppressor of a gene related to the expression of APP,
   in which the microparticles contain miRNA which targets the gene related to the expression of APP.
[5] The gene expression regulator according to [4], in which the microparticles contain at least one kind of the APP suppression-related miRNA group below;
   the APP suppression-related miRNA group:
   hsa-miR-101-3p, hsa-miR-106b-5p, hsa-miR-1229-5p, hsa-miR-1238-3p, hsa-miR-1260b, hsa-miR-1276, hsa-miR-128-3p, hsa-miR-142-3p, hsa-miR-142-5p, hsa-miR-144-3p, hsa-miR-151a-3p, hsa-miR-153-3p, hsa-miR-15a-5p, hsa-miR-15b-5p, hsa-miR-16-5p, hsa-miR-17-5p, hsa-miR-185-5p, hsa-miR-186-5p, hsa-miR-194-5p, hsa-miR-195-5p, hsa-miR-196a-5p, hsa-miR-203a-3p, hsa-miR-20a-5p, hsa-miR-222-3p, hsa-miR-298, hsa-miR-31-5p, hsa-miR-3120-3p, hsa-miR-3132, hsa-miR-323a-3p, hsa-miR-324-5p, hsa-miR-328-3p, hsa-miR-3620-3p, hsa-miR-3646, hsa-miR-369-3p, hsa-miR-373-3p, hsa-miR-374c-5p, hsa-miR-381-3p, hsa-miR-382-5p, hsa-miR-383-5p, hsa-miR-411-3p, hsa-miR-423-3p, hsa-miR-424-3p, hsa-miR-424-5p, hsa-miR-4484, hsa-miR-455-3p, hsa-miR-4786-5p, hsa-miR-484, hsa-miR-490-5p, hsa-miR-497-5p, hsa-miR-500a-5p, hsa-miR-5093, hsa-miR-532-3p, hsa-miR-532-5p, hsa-miR-539-3p, hsa-miR-548f-3p, hsa-miR-551b-5p, hsa-miR-5584-5p, hsa-miR-567, hsa-miR-5696, hsa-miR-582-5p, hsa-miR-6073, hsa-miR-873-5p and hsa-miR-93-5p.
[6] The gene expression regulator according to any one of [1] to [5]
   which is an inhibitor of a gene related to the expression of BACE1,
   in which the microparticles contain miRNA which targets the gene related to the expression of BACE1.
[7] The gene expression regulator according to [6], in which the microparticles contain at least one kind of the BACE1 inhibition-related miRNA group below;
   the BACE1 inhibition-related miRNA group:
   hsa-miR-107, hsa-miR-124-3p, hsa-miR-1267, hsa-miR-128-3p, hsa-miR-129-2-3p, hsa-miR-140-3p, hsa-miR-140-5p, hsa-miR-141-3p, hsa-miR-15a-5p, hsa-miR-16-5p, hsa-miR-17-5p, hsa-miR-195-5p, hsa-miR-200a-3p, hsa-miR-203a-3p, hsa-miR-212-3p, hsa-miR-212-5p, hsa-miR-24-3p, hsa-miR-26b-5p, hsa-miR-27a-3p, hsa-miR-298, hsa-miR-299-3p, hsa-miR-29a-3p, hsa-miR-29b-3p, hsa-miR-29c-3p, hsa-miR-328-3p, hsa-miR-339-5p, hsa-miR-340-5p, hsa-miR-369-3p, hsa-miR-374a-5p, hsa-miR-374b-5p, hsa-miR-374c-5p, hsa-miR-382-5p, hsa-miR-421, hsa-miR-424-5p, hsa-miR-455-3p, hsa-miR-497-5p, hsa-miR-505-3p, hsa-miR-532-5p, hsa-miR-7-5p, hsa-miR-874-3p and hsa-miR-9-5p.
[8] The gene expression regulator according to any one of [1] to [7]
   which is an inhibitor of a gene related to the expression of GSK-3β,
   in which the microparticles contain miRNA which targets the gene related to the expression of GSK-3β.
[9] The gene expression regulator according to [8], in which the microparticles contain at least one kind of the GSK-3β inhibition-related miRNA group below;
   the GSK-3β inhibition-related miRNA group:
   hsa-let-7a-3p, hsa-let-7b-3p, hsa-let-7f-1-3p, hsa-let-7f-2-3p, hsa-miR-101-3p, hsa-miR-1185-1-3p, hsa-miR-1185-2-3p, hsa-miR-124-3p, hsa-miR-128-3p, hsa-miR-129-5p, hsa-miR-132-3p, hsa-miR-137, hsa-miR-140-5p, hsa-miR-142-5p, hsa-miR-144-3p, hsa-miR-150-5p, hsa-miR-155-5p, hsa-miR-15a-5p, hsa-miR-15b-5p, hsa-miR-16-5p, hsa-miR-1910-5p, hsa-miR-195-5p, hsa-miR-199a-5p, hsa-miR-212-3p, hsa-miR-218-5p, hsa-miR-219a-5p, hsa-miR-23a-3p, hsa-miR-24-3p, hsa-miR-26a-5p, hsa-miR-26b-5p, hsa-miR-27a-3p, hsa-miR-28-5p, hsa-miR-29a-3p, hsa-miR-29b-3p, hsa-miR-346, hsa-miR-369-3p, hsa-miR-374a-5p, hsa-miR-374b-5p, hsa-miR-374c-5p, hsa-miR-377-3p, hsa-miR-409-3p, hsa-miR-409-5p, hsa-miR-410-3p, hsa-miR-424-5p, hsa-miR-425-5p, hsa-miR-4465, hsa-miR-497-5p, hsa-miR-582-5p, hsa-miR-6083, hsa-miR-708-5p, hsa-miR-9-5p, hsa-miR-92a-3p, hsa-miR-96-5p and hsa-miR-98-3p.
[10] The gene expression regulator according to any one of [1] to [9], in which the microparticles are of at least one kind of an expression suppressor of a gene related to the expression of APP, an inhibitor of a gene related to the expression of BACE1 and an inhibitor of a gene related to the expression of GSK-3β and contain hsa-miR-16-5p.
[11] The gene expression regulator according to any one of [1] to [10] which is an expression suppressor of the NMDA-activating protein,
   in which the NMDA-activating protein is at least one kind of protein of DLG1, CAMK2D, CAMK2A and CAPN1, and
   the microparticles contain a miRNA group which targets a gene related to the expression of at least one kind of protein of DLG1, CAMK2D, CAMK2A and CAPN1, and the microparticles contain at least one kind of miRNA group among the DLG1 suppression-related miRNA group below, the CAMK2D suppression-related miRNA group below, the CAMK2A suppression-related miRNA group below and the CAPN1 suppression-related miRNA group below;
   the DLG1 suppression-related miRNA group:
      hsa-miR-1-3p, hsa-miR-142-5p, hsa-miR-204-5p, hsa-miR-206, hsa-miR-21-5p, hsa-miR-218-5p, hsa-miR-340-5p and hsa-miR-613,
      the CAMK2D suppression-related miRNA group:
         hsa-let-7a-5p, hsa-miR-101-3p, hsa-miR-129-5p, hsa-miR-139-5p, hsa-miR-144-3p, hsa-miR-145-5p, hsa-miR-185-5p, hsa-miR-203a-3p, hsa-miR-204-5p, hsa-miR-211-5p, hsa-miR-24-3p, hsa-miR-27a-3p, hsa-miR-30a-3p, hsa-miR-31-5p, hsa-miR-361-5p, hsa-miR-421, hsa-miR-484, hsa-miR-494-3p, hsa-miR-505-3p and hsa-miR-7-5p,
         the CAMK2A suppression-related miRNA group:
            hsa-miR-129-5p, hsa-miR-137, hsa-miR-142-5p, hsa-miR-148a-3p, hsa-miR-149-3p, hsa-miR-152-3p, hsa-miR-25-3p, hsa-miR-27a-3p, hsa-miR-32-5p, hsa-miR-338-3p, hsa-miR-340-5p, hsa-miR-363-3p, hsa-miR-3665, hsa-miR-4534, hsa-miR-4665-5p, hsa-miR-4688, hsa-miR-485-5p, hsa-miR-5010-5p, hsa-miR-505-5p, hsa-miR-5698, hsa-miR-625-5p and hsa-miR-92a-3p, and
            the CAPN1 suppression-related miRNA group:
               hsa-miR-1-3p, hsa-miR-124-3p, hsa-miR-140-5p, hsa-miR-17-3p, hsa-miR-22-3p, hsa-miR-34a-5p and hsa-miR-6511b-5p.
[12] The gene expression regulator according to [11],
   in which the microparticles contain all of
   hsa-miR-21-5p of the DLG1 suppression-related miRNA group,
   hsa-let-7a-5p of the CAMK2D suppression-related miRNA group,
   hsa-miR-92a-3p of the CAMK2A suppression-related miRNA group and
   hsa-miR-22-3p of the CAPN1 suppression-related miRNA group.
[13] The gene expression regulator according to any one of [1] to [12]
   which is an expression suppressor of PQBP1,
   in which the microparticles contain miRNA which targets a gene related to the expression of PQBP1.
[14] The gene expression regulator according to [13], in which the microparticles contain at least one kind of the PQBP1 suppression-related miRNA group below;
   the PQBP1 suppression-related miRNA group: hsa-miR-6727-3p and hsa-miR-6727-5p.
[15] The gene expression regulator according to any one of [1] to [14],
   in which the microparticles are microparticles purified and isolated from a culture supernatant of dental pulp-derived stem cells, and
   the gene expression regulator does not contain any component obtained by removing exosomes from the culture supernatant of the dental pulp-derived stem cells.
[16] An agent for preventing or treating Alzheimer's disease containing the gene expression regulator according to any one of [1] to [15].
[17] A method for improving dementia including administering an effective amount of the gene expression regulator according to any one of [1] to [15] or an effective amount of the agent for preventing or treating Alzheimer's disease according to [16] to a subject which has developed amyloid β-related or tau protein-related dementia.

### Advantageous Effects of Invention

According to the invention, a novel gene expression regulator which can regulate the expression of a protein related to amyloid β-related or tau protein-related dementia or brain inflammation such as Alzheimer's disease can be provided.

### Brief Description of Drawings

[FIG. 1] FIG. 1 is a flowchart showing the mechanism of development of Alzheimer's disease.
[FIG. 2] FIG. 2 is a schematic figure of APP processing of the non-amyloidogenic pathway.
[FIG. 3] FIG. 3 is a schematic figure of APP processing of the amyloidogenic pathway.
[FIG. 4] FIG. 4(A) is a schematic figure of induction of the expression of inflammatory genes through the PQBP1-cGAS-STING pathway by cDNA of AIDS virus. FIG. 4(B) is a schematic figure of induction of the expression of inflammatory genes through the PQBP1-cGAS-STING pathway by tau protein.
[FIG. 5] FIG. 5 is a heat map showing the expression levels of miRNAs targeting APP expressed in the exosomes purified from a culture supernatant of dental pulp-derived stem cells (the gene expression regulator of Example 1; SGF) in comparison to the expression levels of the miRNAs in the cerebral cortex.
[FIG. 6] FIG. 6 is a heat map showing the expression levels of miRNAs targeting β secretase (BASE1) expressed in the exosomes purified from a culture supernatant of dental pulp-derived stem cells (the gene expression regulator of Example 1; SGF) in comparison to the expression levels of the miRNAs in the cerebral cortex.
[FIG. 7] FIG. 7 is a heat map showing the expression levels of miRNAs targeting DLG1 expressed in the exosomes purified from a culture supernatant of dental pulp-derived stem cells (the gene expression regulator of Example 1; SGF) in comparison to the expression levels of the miRNAs in the cerebral cortex.
[FIG. 8] FIG. 8 is a heat map showing the expression levels of miRNAs targeting CAMK2D expressed in the exosomes purified from a culture supernatant of dental pulp-derived stem cells (the gene expression regulator of Example 1; SGF) in comparison to the expression levels of the miRNAs in the cerebral cortex.
[FIG. 9] FIG. 9 is a heat map showing the expression levels of miRNAs targeting CAMK2A expressed in the exosomes purified from a culture supernatant of dental pulp-derived stem cells (the gene expression regulator of Example 1; SGF) in comparison to the expression levels of the miRNAs in the cerebral cortex.
[FIG. 10] FIG. 10 is a heat map showing the expression levels of miRNAs targeting CAPN1 expressed in the exosomes purified from a culture supernatant of dental pulp-derived stem cells (the gene expression regulator of Example 1; SGF) in comparison to the expression levels of the miRNAs in the cerebral cortex.
[FIG. 11] FIG. 11 is a heat map showing the expression levels of miRNAs targeting GSK-3β expressed in the exosomes purified from a culture supernatant of dental pulp-derived stem cells (the gene expression regulator of Example 1; SGF) in comparison to the expression levels of the miRNAs in the cerebral cortex.
[FIG. 12] FIG. 12 is a heat map showing the expression levels of miRNAs targeting PQBP1 expressed in the exosomes purified from a culture supernatant of dental pulp-derived stem cells (the gene expression regulator of Example 1; SGF) in comparison to the expression levels of the miRNAs in the cerebral cortex.
[FIG. 13] FIG. 13 is a heat map showing the expression levels of amyloid β- or tau protein-related miRNAs expressed in the exosomes purified from a culture supernatant of dental pulp-derived stem cells (the gene expression regulator of Example 1; SGF) in comparison to the expression levels of the miRNAs in the cerebral cortex (first page).
[FIG. 13-2] FIG. 13-2 is the rest of the heat map of FIG. 13 (second page).
[FIG. 14] FIG. 14 is a bar graph showing the expression levels of APP gene in Test Example 4.
[FIG. 15] FIG. 15 is a bar graph showing the expression levels of BASE1 gene in Test Example 4.
[FIG. 16] FIG. 16 is a bar graph showing the expression levels of CAMK2D gene in Test Example 4.
[FIG. 17] FIG. 17 is a bar graph showing the expression levels of GSK-3β gene in Test Example 4.
[FIG. 18] FIG. 18 is a bar graph showing the expression levels of PQBP1 gene in Test Example 4.
[FIG. 19] FIG. 19 is a bar graph showing the expression levels of miRNAs in Test Example 5.

### Description of Embodiments

The invention is explained in detail below. The constituent features described below are sometimes explained based on typical embodiments and specific examples, but the invention is not limited to such embodiments. Here, a numerical range expressed using "to" in the present description means the range including the numerical values before and after "to" as the lower limit and the upper limit.

### [Gene Expression Regulator]

The gene expression regulator of the invention contains microparticles containing miRNA which targets a gene (also simply called a target) related to the expression of at least one kind of protein of amyloid precursor protein (APP), β-secretase (BACE1), an NMDA-activating protein and glycogen synthase kinase-3β (GSK-3β).

The gene expression regulator of the invention can regulate the expression of a gene related to a protein related to amyloid β-related or tau protein-related dementia or brain inflammation. As a result, the gene expression regulator of the invention can also preferably improve amyloid β-related or tau protein-related dementia or brain inflammation and can also more preferably prevent or treat the same. The treatment means to alleviate a symptom of amyloid β-related or tau protein-related dementia or brain inflammation and does not necessarily achieve complete cure. The treatment of amyloid β-related or tau protein-related dementia is preferably to alleviate a symptom of the cognitive function or the behavior.

Preferable aspects of the gene expression regulator of the invention are explained below.

### <Definition of Dementia>

In the invention, dementia generally means the state with an acquired decrease in the cognitive function.

Examples of the disease type of dementia include Alzheimer-type dementia (AD), vascular dementia (VaD), dementia with Lewy body (DLB, including Parkinson's disease with dementia PDD), frontotemporal lobar degeneration (including frontotemporal dementia (FTD), semantic dementia (SD) and progressive nonfluent aphasia (PNFA)), alcohol-induced dementia, mixed type, mild cognitive impairment (MCI), and early onset dementia.

The dementia as the subject of administration of the gene expression regulator of the invention is preferably amyloid β-related or tau protein-related dementia. The amyloid β-related or tau protein-related dementia is not particularly restricted and is a symptom group in which tau accumulation, or a so-called tau patch, is caused. Specifically, the dementia as the subject of administration of the gene expression regulator of the invention is preferably Alzheimer-type dementia, frontotemporal dementia (FTD or bvFTD), semantic dementia (SD) or progressive nonfluent aphasia (PNFA). FTD includes Pick's disease.

The dementia as the subject of administration of the gene expression regulator of the invention is more preferably Alzheimer-type dementia.

### <Mechanism of Development of Alzheimer's Disease>

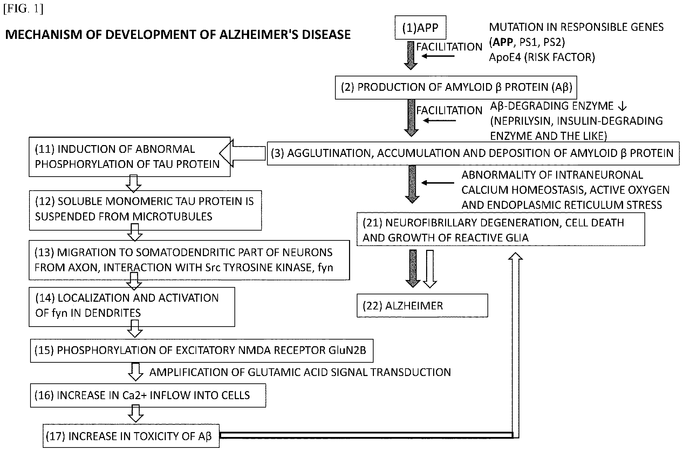

A flowchart showing the mechanism of development of Alzheimer's disease is shown in FIG. 1.

First, the flowchart with the black arrows in FIG. 1 is explained.
(1) Production of amyloid precursor protein (APP). Further, mutation in the responsible genes (APP, PS1 and PS2) and ApoE4 (risk factor) facilitate APP.
(2) Production of amyloid β protein (Aβ). Further, suppression of Aβ-degrading enzymes (neprilysin, insulin-degrading enzyme and the like) facilitates the production of Aβ.
(3) Agglutination, accumulation and deposition of amyloid β protein. Further, abnormality of intraneuronal calcium homeostasis, active oxygen and endoplasmic reticulum stress advance the agglutination, the accumulation and the deposition of Aβ.
(21) Neurofibrillary degeneration, cell death and growth of reactive glia.
(22) Development of Alzheimer's disease.

Next, the flowchart with the white arrows in FIG. 1 is explained.
(11) Induction of abnormal phosphorylation of tau protein advances due to (3) agglutination, accumulation and deposition of amyloid β protein.
(12) Soluble monomeric tau protein is suspended from microtubules.
(13) Migration to the somatodendritic part of neurons from the axon. Monomeric tau protein interacts with Src tyrosine kinase, fyn.
(14) Localization and activation of fyn in dendrites.
(15) Phosphorylation of excitatory NMDA receptor GluN2B. Amplification of glutamic acid signal transduction advances the phosphorylation of excitatory NMDA receptor GluN2B.
(16) Increase in the Ca²⁺ inflow into cells.
(17) Increase in the toxicity of Aβ.
(21) Neurofibrillary degeneration, cell death and growth of reactive glia.
(22) Development of Alzheimer's disease.

### (APP Processing)

In connection with (2) production of amyloid β protein (Aβ) in FIG. 1, the processing of amyloid precursor protein (APP) is explained.

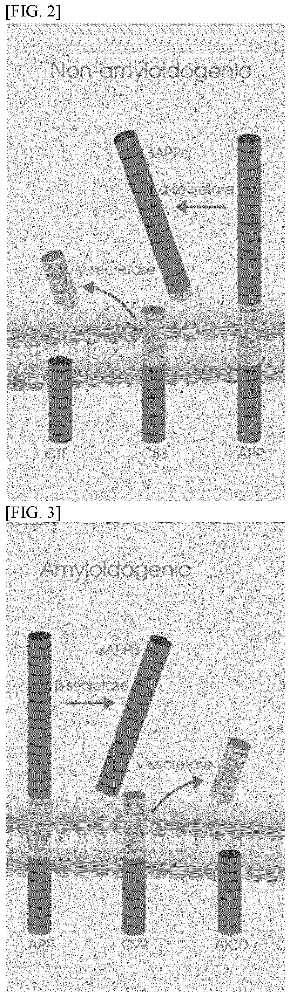

FIG. 2 is a schematic figure of APP processing of the non-amyloidogenic pathway.

First, when APP is cleaved by α secretase, C83 (a fragment composed of the 83 amino acids at the C terminal) remains in the cell membrane, and sAPPα (a fragment composed of the extracellular domain at the N terminal) is released from the cells.

Next, when C83 is cleaved by γ secretase, P3 peptide and CTF are produced. As a result, Aβ is not produced.

On the other hand, FIG. 3 is a schematic figure of APP processing of the amyloidogenic pathway.

First, APP is cleaved by β-secretase (BACE1), and C99 (the 99-amino-acid fragment at the C terminal) remains in the cell membrane. sAPPβ (the extracellular domain at the N terminal) is released from the cells.

Next, C99 is cleaved by γ secretase, and Aβ peptide (Aβ1-40 or Aβ1-42) is formed.

Here, γ-secretase is composed of Presenilin 1 (PS1) or Presenilin 2 (PS2), Nicastrin, APH-1 and PEN2.

### <Agent for Treating Alzheimer's Disease and Mechanism of Action>

The gene expression regulator of the invention can preferably improve Alzheimer's disease in particular, of dementia. The agent for treating Alzheimer's disease and the mechanism of action thereof are explained below together with the mechanism of action of the gene expression regulator of the invention.

The agent for treating Alzheimer's disease and the mechanism of action thereof may be as follows.

### (a) Inhibitor of Acetylcholine-Degrading Enzyme

Acetylcholine is a substance which is released from the synaptic sites of the telodendria of a neuron and transmits the signal to the next neuron, and the number of cells producing acetylcholine is decreased in the brain of Alzheimer-type dementia. Using an inhibitor of an acetylcholine-degrading enzyme (donepezil hydrochloride or the like), the degradation and the loss of acetylcholine in the synapses is suppressed, and the amount of acetylcholine is increased.

Here, the inhibitor of acetylcholine-degrading enzyme is poorly relevant to the gene expression regulator of the invention.

### (b) Nicotinic Receptor-Stimulating Agent

Signal transduction of acetylcholine to a nicotinic receptor is caused by binding to an acetylcholine receptor. Acetylcholine receptors include nicotinic receptors (also called nicotinic acetylcholine receptors) and muscarinic receptors. A nicotinic receptor is a pentamer composed of subunits and functions as an ion channel when acetylcholine binds thereto. In the brain of Alzheimer-type dementia, the number of nicotinic receptors is decreased. Galantamine hydrobromide or the like binds to a different site to that of acetylcholine and enhances the function of the nicotinic receptor (APL action).

Here, the nicotinic receptor-stimulating agent is poorly relevant to the gene expression regulator of the invention.

### (c) NMDA Receptor Inhibitor

By suppressing the activation of NMDA receptors induced by an increase in Aβ or tau, cell death caused by excessive excitation of neurons is prevented. NMDA is involved in memory, learning and brain ischemia. Proteins which regulate NMDA include DLG1, CAMK2D, CAMK2A, CAPN1 and EPHB2.

When the microparticles contain miRNA which targets a gene related to the expression of an NMDA-activating protein, the gene expression regulator of the invention functions as an NMDA receptor inhibitor. In particular, the gene expression regulator of the invention functions as at least one kind of NMDA receptor inhibitor of a DLG1 expression suppressor, a CAMK2D expression suppressor, a CAMK2A expression suppressor and a CAPN1 expression suppressor.

### (d) BACE1 Inhibitor

BACE1, which is an enzyme that produces Aβ, is inhibited, and the production of Aβ is suppressed.

When the microparticles contain miRNA which targets a gene related to the expression of β-secretase (BACE1), the gene expression regulator of the invention functions as a BACE1 inhibitor.

### (e) GSK-3β Inhibitor

Glycogen synthase kinase-3β (GSK-3β) is an enzyme which phosphorylates proteins with various chains and has various important functions in maintenance of the lives of and regulation of normal cells. When GSK-3β is enhanced abnormally, deposition of Aβ in the brain and accumulation of tau protein in neurons are promoted. A GSK-3β inhibitor suppresses deposition of Aβ and abnormal phosphorylation of tau protein (described in Table 1 below, see Rinsho Shinkeigaku (Clinical Neurology), 54(12), 1178-1180, 2014).

When the microparticles contain miRNA which targets a gene related to the expression of glycogen synthase kinase-3β (GSK-3β), the gene expression regulator of the invention functions as a GSK-3β inhibitor.

**[Table 1]**

| Target | Compound | Mechanism of Action | Subject | Clinical Trial (phase) | Result |
|---|---|---|---|---|---|
| Excessive Phosphorylation | Lithium | GSK3β inhibition | aMCI | 2 | Improvement of cognitive function |
| | | | | | Decrease in CSF p-tau |
| | Tideglusib | GSK3β inhibition | Mild to moderate AD | 2 | Tendency towards improvement of cognitive function |
| Agglutination | Methylene blue | Tau agglutination inhibition | Mild to moderate AD | 2 | Improvement of cognitive function |
| | | | | | Improvement of brain blood flow |
| | | | | | Increase in use of brain saccharides |
| Tau | AADvac1 | Active immunotherapy | Mild to moderate AD | 1 | Under test |

| | | | | | |
|---|---|---|---|---|---|
| Typical tau disease-modifying drugs which have been advanced to clinical trials so far are shown. aMCI; amnestic mild cognitive impairment, GSK; Glycogen synthase kinase, AD; Alzheimer's disease, CSF; cerebral spinal fluid. | | | | | |

### (f) APP Inhibitor

An APP inhibitor inhibits the production of amyloid precursor protein (APP) and suppresses the production of APP.

When the microparticles contain miRNA which targets a gene related to the expression of amyloid precursor protein (APP), the gene expression regulator of the invention functions as an APP inhibitor.

### <Brain Inflammation>

The gene expression regulator of the invention can preferably improve brain inflammation. The mechanism of action of brain inflammation is explained below together with the mechanism of action of the gene expression regulator of the invention.

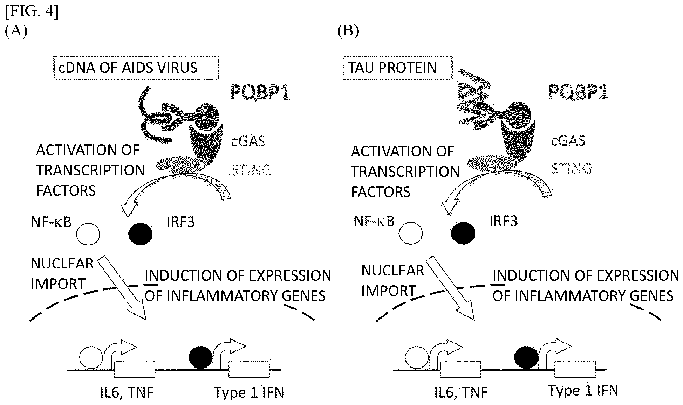

Tau protein, which involves in pathology of neurodegenerative diseases including Alzheimer's disease as well as frontotemporal lobar degeneration, Parkinson's disease and Huntington's disease, is recognized by PQBP1, which is known as the intracellular receptor for AIDS virus, in the brain microglia and induces brain inflammation. Specifically, tau activates microglia through the PQBP1-cGAC-STING pathway and promotes brain inflammation (Nature Communications volume 12, Article number: 6565 (2021)). FIG. 4(A) is a schematic figure of induction of the expression of inflammatory genes through the PQBP1-cGAS-STING pathway by the cDNA of AIDS virus. FIG. 4(B) is a schematic figure of induction of the expression of inflammatory genes through the PQBP1-cGAS-STING pathway by tau protein.

Accordingly, when the microparticles contain miRNA which targets a gene related to the expression of polyglutamine-binding protein-1 (PQBP1), the gene expression regulator of the invention functions as a PQBP1 suppressor. As a result, the gene expression regulator of the invention can improve tau protein-related dementia (in particular, Alzheimer's disease) or brain inflammation.

### <Microparticles>

The miRNA which is an active ingredient of the gene expression regulator of the invention is contained in the microparticles.

The microparticles used as the gene expression regulator of the invention are derived from dental pulp-derived stem cells or the like, for example, through secretion, emergence, dispersion or the like from mesenchymal stem cells such as the dental pulp-derived stem cells and are exuded or released or fall into a cell culture medium. The microparticles are preferably contained in a culture supernatant of dental pulp-derived stem cells or the like and are more preferably microparticles derived from a culture supernatant of dental pulp-derived stem cells. Here, however, the microparticles derived from a culture supernatant of dental pulp-derived stem cells do not necessarily have to be obtained from a culture supernatant of dental pulp-derived stem cells. For example, microparticles inside dental pulp-derived stem cells which have been isolated by any method can be considered as microparticles derived from a culture supernatant of dental pulp-derived stem cells as long as the microparticles are the same as the microparticles which can be isolated from a culture supernatant of dental pulp-derived stem cells.

The microparticles derived from a culture supernatant of dental pulp-derived stem cells or the like may be used in the state of being contained in the culture supernatant or the state of being purified from the culture supernatant. The microparticles are preferably microparticles purified from the culture supernatant.

The origin of the microparticles can be identified by a known method. For example, the stem cells, such as dental pulp-derived stem cells, adipose-derived stem cells, bone marrow-derived stem cells and umbilical cord-derived stem cells, from which the microparticles are derived can be determined by the method described in J Stem Cell Res Ther (2018) 8:2. Specifically, based on the miRNA pattern of the microparticles, the origin of the microparticles can be identified.

### (miRNA)

In the invention, the microparticles contain miRNA which targets a gene related to the expression of at least one kind of protein of amyloid precursor protein (APP), β-secretase (BACE1), an NMDA-activating protein, glycogen synthase kinase-3β (GSK-3β) and polyglutamine-binding protein-1 (PQBP1).

In the invention, the miRNAs (microRNAs) are, for example, RNA molecules of 21 to 25 bases (nucleotides). The miRNAs can regulate gene expression through degradation of the target gene (target) mRNA or suppression in the decoding stage.

In the invention, the miRNAs may be single stranded (monomer) or double stranded (dimer). Moreover, in the invention, the miRNAs are preferably mature-form miRNAs which have been cleaved by a ribonuclease such as dicer.

Here, the sequences of the miRNAs described in the present description, such as hsa-miR-16-5p, are registered with accession numbers in known databases (for example, miRBase database), and one skilled in the art can determine the sequences unambiguously. For example, the accession number of hsa-miR-16-5p is MIMAT0000069, and the sequence is registered in miRBase database. The accession numbers of the miRNAs are not shown below.

The miRNAs in the present description, however, include variants in which around one to five bases are different in a mature-form miRNA, such as hsa-miR-16-5p. Moreover, each miRNA in the present description includes a polynucleotide having a base sequence having identity with the base sequence of the miRNA (for example, hsa-miR-16-5p) or a polynucleotide which has a complementary base sequence thereof and which has the function of the miRNA in the invention. The "identity" is the degree of identity obtained when the sequences to be compared are appropriately aligned and means the incidence rate (%) of the accurately matching amino acids in the sequences. The sequences can be aligned, for example, using any algorithm such as BLAST. The identity is, for example, 80%, 85%, 90%, 91%, 92%, 93%, 94%, 95%, 96%, 97%, 98% or about 99%. The polynucleotide having a base sequence having identity may have, for example, point mutation, deletion and/or addition in the base sequence of the miRNA. The number of bases of the point mutation or the like is, for example, one to five, one to three, one or two or one. Moreover, the polynucleotide having a complementary base sequence includes, for example, a polynucleotide which hybridizes to the polynucleotide having the base sequence of the miRNA under stringent conditions and which has the function of the miRNA in the invention. The stringent conditions are not particularly limited but are, for example, the conditions described in [0028] of JP2017-184642A. The contents of the publication are incorporated in the present description by reference.

In the invention, the microparticles preferably contain the miRNA which targets a gene related to the expression of at least one kind of protein of APP, BACE1, an NMDA-activating protein, GSK-3β and PQBP1 at a higher concentration than that of a culture supernatant of dental pulp-derived stem cells.

Preferable aspects of the miRNA contained in the microparticles are explained below.

### (1) Expression Suppressor of APP

The gene expression regulator of the invention is preferably an expression suppressor of APP. In this case, the microparticles preferably contain miRNA which targets a gene related to the expression of APP.

In the invention, the microparticles more preferably contain at least one kind of the APP suppression-related miRNA group (63 kinds) below.

The APP suppression-related miRNA group:
hsa-miR-101-3p, hsa-miR-106b-5p, hsa-miR-1229-5p, hsa-miR-1238-3p, hsa-miR-1260b, hsa-miR-1276, hsa-miR-128-3p, hsa-miR-142-3p, hsa-miR-142-5p, hsa-miR-144-3p, hsa-miR-151a-3p, hsa-miR-153-3p, hsa-miR-15a-5p, hsa-miR-15b-5p, hsa-miR-16-5p, hsa-miR-17-5p, hsa-miR-185-5p, hsa-miR-186-5p, hsa-miR-194-5p, hsa-miR-195-5p, hsa-miR-196a-5p, hsa-miR-203a-3p, hsa-miR-20a-5p, hsa-miR-222-3p, hsa-miR-298, hsa-miR-31-5p, hsa-miR-3120-3p, hsa-miR-3132, hsa-miR-323a-3p, hsa-miR-324-5p, hsa-miR-328-3p, hsa-miR-3620-3p, hsa-miR-3646, hsa-miR-369-3p, hsa-miR-373-3p, hsa-miR-374c-5p, hsa-miR-381-3p, hsa-miR-382-5p, hsa-miR-383-5p, hsa-miR-411-3p, hsa-miR-423-3p, hsa-miR-424-3p, hsa-miR-424-5p, hsa-miR-4484, hsa-miR-455-3p, hsa-miR-4786-5p, hsa-miR-484, hsa-miR-490-5p, hsa-miR-497-5p, hsa-miR-500a-5p, hsa-miR-5093, hsa-miR-532-3p, hsa-miR-532-5p, hsa-miR-539-3p, hsa-miR-548f-3p, hsa-miR-551b-5p, hsa-miR-5584-5p, hsa-miR-567, hsa-miR-5696, hsa-miR-582-5p, hsa-miR-6073, hsa-miR-873-5p and hsa-miR-93-5p.

The microparticles preferably contain five kinds or more of the APP suppression-related miRNA group, more preferably contain 10 kinds or more, particularly preferably contain 20 kinds or more or further particularly preferably contain 30 kinds or more.

The microparticles preferably contain any one kind of hsa-miR-101-3p, hsa-miR-153-3p, hsa-miR-16-5p, hsa-miR-222-3p and hsa-miR-93-5p, of the APP suppression-related miRNA group or particularly preferably contain at least hsa-miR-16-5p.

The microparticles contain at least one kind of the APP suppression-related miRNA group at a Log2Ratio of the read count number obtained by analysis using IMOTA of preferably 4.0 or more, more preferably 10.0 or more, particularly preferably 15.0 or more. The microparticles preferably contain all of hsa-miR-101-3p, hsa-miR-153-3p, hsa-miR-16-5p, hsa-miR-222-3p and hsa-miR-93-5p at a Log2Ratio of the read count number obtained by analysis using IMOTA of 4.0 or more, more preferably contain all of hsa-miR-101-3p, hsa-miR-16-5p, hsa-miR-222-3p and hsa-miR-93-5p at 10.0 or more or particularly preferably contain hsa-miR-16-5p at 15.0 or more.

Here, hsa-miR-16-5p included in the APP suppression-related miRNA group is also an inhibitor of a gene related to the expression of BACE1 and an inhibitor of a gene related to the expression of GSK-3β. That is, in the invention, the microparticles are preferably of at least one kind of an expression suppressor of a gene related to the expression of APP, an inhibitor of a gene related to the expression of BACE1 and an inhibitor of a gene related to the expression of GSK-3β and contain hsa-miR-16-5p.

Further, the microparticles are more preferably an expression suppressor of a gene related to the expression of APP, an inhibitor of a gene related to the expression of BACE1 and an inhibitor of a gene related to the expression of GSK-3β and contain hsa-miR-16-5p.

In the gene expression regulator of the invention, the expression level of hsa-miR-16-5p is preferably 1.1 times or more, more preferably 1.5 times or more, particularly preferably two times or more, compared to that in exosomes obtained from a culture supernatant of adipose-derived stem cells or exosomes obtained from a culture supernatant of umbilical cord-derived stem cells.

When the gene expression regulator of the invention is an expression suppressor of APP gene, the gene expression regulator can preferably suppress the expression of APP gene in any cells to 0.8 times or less of the normal level (of untreated cells), can more preferably suppress to 0.6 times or less or can particularly preferably suppress to 0.4 times or less.

### (2) BACE1 Inhibitor

The gene expression regulator of the invention is preferably a BACE1 inhibitor. In this case, the microparticles preferably contain miRNA which targets a gene related to the expression of BACE1.

In the invention, the microparticles more preferably contain at least one kind of the BACE1 inhibition-related miRNA group (50 kinds) below.

The BACE1 inhibition-related miRNA group:
hsa-miR-107, hsa-miR-124-3p, hsa-miR-1267, hsa-miR-128-3p, hsa-miR-129-2-3p, hsa-miR-140-3p, hsa-miR-140-5p, hsa-miR-141-3p, hsa-miR-15a-5p, hsa-miR-16-5p, hsa-miR-17-5p, hsa-miR-195-5p, hsa-miR-200a-3p, hsa-miR-203a-3p, hsa-miR-212-3p, hsa-miR-212-5p, hsa-miR-24-3p, hsa-miR-26b-5p, hsa-miR-27a-3p, hsa-miR-298, hsa-miR-299-3p, hsa-miR-29a-3p, hsa-miR-29b-3p, hsa-miR-29c-3p, hsa-miR-328-3p, hsa-miR-339-5p, hsa-miR-340-5p, hsa-miR-369-3p, hsa-miR-374a-5p, hsa-miR-374b-5p, hsa-miR-374c-5p, hsa-miR-382-5p, hsa-miR-421, hsa-miR-424-5p, hsa-miR-455-3p, hsa-miR-497-5p, hsa-miR-505-3p, hsa-miR-532-5p, hsa-miR-7-5p, hsa-miR-874-3p and hsa-miR-9-5p.

The microparticles preferably contain five kinds or more of the BACE1 inhibition-related miRNA group, more preferably contain 10 kinds or more, particularly preferably contain 20 kinds or more or further particularly preferably contain 30 kinds or more.

The microparticles preferably contain any one kind of hsa-miR-101-3p, hsa-miR-16-5p and hsa-miR-29a-3p of the BACE1 inhibition-related miRNA group or particularly preferably contain at least hsa-miR-16-5p.

The microparticles contain at least one kind of the BACE1 inhibition-related miRNA group at a Log2Ratio of the read count number obtained by analysis using IMOTA of preferably 4.0 or more, more preferably 10.0 or more, particularly preferably 15.0 or more. The microparticles preferably contain all of hsa-miR-101-3p, hsa-miR-16-5p and hsa-miR-29a-3p at a Log2Ratio of the read count number obtained by analysis using IMOTA of 4.0 or more, more preferably contain all of hsa-miR-101-3p, hsa-miR-16-5p and hsa-miR-29a-3p at 10.0 or more or particularly preferably contain hsa-miR-16-5p at 15.0 or more.

When the gene expression regulator of the invention is an expression suppressor of BACE1 gene, the gene expression regulator can preferably suppress the expression of BACE1 gene in any cells to 0.8 times or less of the normal level (of untreated cells), can more preferably suppress to 0.75 times or less or can particularly preferably suppress to 0.7 times or less.

### (3) Expression Suppressor of NMDA-Activating Gene

The gene expression regulator of the invention is preferably an expression suppressor of an NMDA-activating gene. In this case, the NMDA-activating protein is at least one kind of protein of DLG1, CAMK2D, CAMK2A and CAPN1, and the microparticles preferably contain miRNA which targets a gene related to the expression of at least one kind of protein of DLG1, CAMK2D, CAMK2A and CAPN1.

In the invention, the microparticles more preferably contain at least one kind of miRNA group of the DLG1 suppression-related miRNA group below, the CAMK2D suppression-related miRNA group below, the CAMK2A suppression-related miRNA group below and the CAPN1 suppression-related miRNA group below (51 kinds in total).

The DLG1 suppression-related miRNA group:
hsa-miR-1-3p, hsa-miR-142-5p, hsa-miR-204-5p, hsa-miR-206, hsa-miR-21-5p, hsa-miR-218-5p, hsa-miR-340-5p and hsa-miR-613.

The CAMK2D suppression-related miRNA group:
hsa-let-7a-5p, hsa-miR-101-3p, hsa-miR-129-5p, hsa-miR-139-5p, hsa-miR-144-3p, hsa-miR-145-5p, hsa-miR-185-5p, hsa-miR-203a-3p, hsa-miR-204-5p, hsa-miR-211-5p, hsa-miR-24-3p, hsa-miR-27a-3p, hsa-miR-30a-3p, hsa-miR-31-5p, hsa-miR-361-5p, hsa-miR-421, hsa-miR-484, hsa-miR-494-3p, hsa-miR-505-3p and hsa-miR-7-5p.

The CAMK2A suppression-related miRNA group:
hsa-miR-129-5p, hsa-miR-137, hsa-miR-142-5p, hsa-miR-148a-3p, hsa-miR-149-3p, hsa-miR-152-3p, hsa-miR-25-3p, hsa-miR-27a-3p, hsa-miR-32-5p, hsa-miR-338-3p, hsa-miR-340-5p, hsa-miR-363-3p, hsa-miR-3665, hsa-miR-4534, hsa-miR-4665-5p, hsa-miR-4688, hsa-miR-485-5p, hsa-miR-5010-5p, hsa-miR-505-5p, hsa-miR-5698, hsa-miR-625-5p and hsa-miR-92a-3p.

The CAPN1 suppression-related miRNA group:
hsa-miR-1-3p, hsa-miR-124-3p, hsa-miR-140-5p, hsa-miR-17-3p, hsa-miR-22-3p, hsa-miR-34a-5p and hsa-miR-6511b-Sp.

The microparticles preferably contain five kinds or more of the DLG1 suppression-related miRNA group, the CAMK2D suppression-related miRNA group, the CAMK2A suppression-related miRNA group and the CAPN1 suppression-related miRNA group, more preferably contain 10 kinds or more, particularly preferably contain 20 kinds or more or further particularly preferably contain 30 kinds or more.

The microparticles particularly preferably contain all of
hsa-miR-21-5p of the DLG1 suppression-related miRNA group,
hsa-let-7a-5p of the CAMK2D suppression-related miRNA group,
hsa-miR-92a-3p of the CAMK2A suppression-related miRNA group and
hsa-miR-22-3p of the CAPN1 suppression-related miRNA group.

The microparticles contain at least one kind of the DLG1 suppression-related miRNA group, the CAMK2D suppression-related miRNA group, the CAMK2A suppression-related miRNA group and the CAPN1 suppression-related miRNA group at a Log2Ratio of the read count number obtained by analysis using IMOTA of preferably 4.0 or more, more preferably 10.0 or more, particularly preferably 15.0 or more. The microparticles preferably contain all of hsa-miR-21-5p, hsa-let-7a-5p, hsa-miR-92a-3p and hsa-miR-22-3p at a Log2Ratio of the read count number obtained by analysis using IMOTA of 4.0 or more, more preferably contain all of hsa-miR-21-5p, hsa-let-7a-5p, hsa-miR-92a-3p and hsa-miR-22-3p at 10.0 or more or particularly preferably contain hsa-miR-21-5p, hsa-let-7a-5p and hsa-miR-92a-3p at 15.0 or more.

In the gene expression regulator of the invention, the expression level of hsa-miR-21-5p is preferably 1.1 times or more, more preferably 1.5 times or more, particularly preferably two times or more, further particularly preferably five times or more, compared to that in exosomes obtained from a culture supernatant of adipose-derived stem cells or exosomes obtained from a culture supernatant of umbilical cord-derived stem cells.

In the gene expression regulator of the invention, the expression level of hsa-let-7a-5p is preferably 1.1 times or more, more preferably 1.5 times or more, compared to that in exosomes obtained from a culture supernatant of adipose-derived stem cells. Moreover, in the gene expression regulator of the invention, the expression level of hsa-let-7a-5p preferably exceeds 1.0 time or is more preferably 1.05 times or more, compared to that in exosomes obtained from a culture supernatant of umbilical cord-derived stem cells.

In the gene expression regulator of the invention, the expression level of hsa-miR-92a-3p is preferably 1.1 times or more, more preferably 1.5 times or more, particularly preferably two times or more, further particularly preferably four times or more, compared to that in exosomes obtained from a culture supernatant of adipose-derived stem cells or exosomes obtained from a culture supernatant of umbilical cord-derived stem cells.

In the gene expression regulator of the invention, the expression level of hsa-miR-22-3p is preferably 1.1 times or more, more preferably 1.5 times or more, particularly preferably two times or more, compared to that in exosomes obtained from a culture supernatant of adipose-derived stem cells or exosomes obtained from a culture supernatant of umbilical cord-derived stem cells.

When the gene expression regulator of the invention is an expression suppressor of an NMDA-activating gene (preferably CAMK2D gene), the gene expression regulator can preferably suppress the expression of the NMDA-activating gene in any cells to 0.8 times or less of the normal level (of untreated cells), can more preferably suppress to 0.5 times or less or can particularly preferably suppress to 0.3 times or less.

### (4) GSK-3β Inhibitor

The gene expression regulator of the invention is preferably a GSK-3β inhibitor. In this case, the microparticles preferably contain miRNA which targets a gene related to the expression of GSK-3β.

The microparticles more preferably contain at least one kind of the GSK-3β inhibition-related miRNA group (54 kinds) below.

The GSK-3β inhibition-related miRNA group:
hsa-let-7a-3p, hsa-let-7b-3p, hsa-let-7f-1-3p, hsa-let-7f-2-3p, hsa-miR-101-3p, hsa-miR-1185-1-3p, hsa-miR-1185-2-3p, hsa-miR-124-3p, hsa-miR-128-3p, hsa-miR-129-5p, hsa-miR-132-3p, hsa-miR-137, hsa-miR-140-5p, hsa-miR-142-5p, hsa-miR-144-3p, hsa-miR-150-5p, hsa-miR-155-5p, hsa-miR-15a-5p, hsa-miR-15b-5p, hsa-miR-16-5p, hsa-miR-1910-5p, hsa-miR-195-5p, hsa-miR-199a-5p, hsa-miR-212-3p, hsa-miR-218-5p, hsa-miR-219a-5p, hsa-miR-23a-3p, hsa-miR-24-3p, hsa-miR-26a-5p, hsa-miR-26b-5p, hsa-miR-27a-3p, hsa-miR-28-5p, hsa-miR-29a-3p, hsa-miR-29b-3p, hsa-miR-346, hsa-miR-369-3p, hsa-miR-374a-5p, hsa-miR-374b-5p, hsa-miR-374c-5p, hsa-miR-377-3p, hsa-miR-409-3p, hsa-miR-409-5p, hsa-miR-410-3p, hsa-miR-424-5p, hsa-miR-425-5p, hsa-miR-4465, hsa-miR-497-5p, hsa-miR-582-5p, hsa-miR-6083, hsa-miR-708-5p, hsa-miR-9-5p, hsa-miR-92a-3p, hsa-miR-96-5p and hsa-miR-98-3p.

The microparticles preferably contain five kinds or more of the GSK-3β inhibition-related miRNA group, more preferably contain 10 kinds or more, particularly preferably contain 20 kinds or more or further particularly preferably contain 30 kinds or more.

The microparticles preferably contain any one kind of hsa-miR-16-5p and hsa-miR-29a-3p of the GSK-3β inhibition-related miRNA group or particularly preferably contain at least hsa-miR-16-5p.

The microparticles contain at least one kind of the GSK-3β inhibition-related miRNA group at a Log2Ratio of the read count number obtained by analysis using IMOTA of preferably 4.0 or more, more preferably 10.0 or more, particularly preferably 15.0 or more. The microparticles preferably contain both hsa-miR-16-5p and hsa-miR-29a-3p at a Log2Ratio of the read count number obtained by analysis using IMOTA of 4.0 or more, more preferably contain both hsa-miR-16-5p and hsa-miR-29a-3p at 10.0 or more or particularly preferably contain hsa-miR-16-5p at 15.0 or more.

When the gene expression regulator of the invention is an inhibitor of GSK-3β gene, the gene expression regulator can preferably suppress the expression of GSK-3β gene in any cells to 0.8 times or less of the normal level (of untreated cells), can more preferably suppress to 0.75 times or less or can particularly preferably suppress to 0.7 times or less.

### (5) Expression Suppressor of PQBP1

The gene expression regulator of the invention is preferably an expression suppressor of PQBP1. In this case, the microparticles more preferably contain miRNA which targets a gene related to the expression of PQBP1. As shown in Nature Communications volume 12, Article number: 6565 (2021), miRNA which suppresses PQBP1 involved in the activation of tau may help treating tau disease (Alzheimer's disease or brain inflammation).

The microparticles particularly preferably contain at least one kind of the PQBP1 suppression-related miRNA group below or further particularly preferably contain the two kinds.

The PQBP1 suppression-related miRNA group: hsa-miR-6727-3p and hsa-miR-6727-5p.

The microparticles more preferably contain at least hsa-miR-6727-3p of the PQBP1 suppression-related miRNA group or particularly preferably contain both of hsa-miR-6727-3p and hsa-miR-6727-5p.

The microparticles contain at least one kind of the PQBP1 suppression-related miRNA group at a Log2Ratio of the read count number obtained by analysis using IMOTA of preferably 1.0 or more, more preferably 2.0 or more, particularly preferably 3.0 or more. The microparticles preferably contain both hsa-miR-6727-3p and hsa-miR-6727-5p at a Log2Ratio of the read count number obtained by analysis using IMOTA of 2.0 or more, more preferably contain both of hsa-miR-6727-3p and hsa-miR-6727-5p at 3.0 or more or particularly preferably contain hsa-miR-6727-3p at 4.0 or more.

In the gene expression regulator of the invention, the expression level of hsa-miR-6727-3p is preferably 1.1 times or more, more preferably 1.5 times or more, particularly preferably two times or more, compared to that in exosomes obtained from a culture supernatant of adipose-derived stem cells or exosomes obtained from a culture supernatant of umbilical cord-derived stem cells.

When the gene expression regulator of the invention is an expression suppressor of PQBP1 gene, the gene expression regulator can preferably suppress the expression of PQBP1 gene in any cells to 0.8 times or less of the normal level (of untreated cells), can more preferably suppress to 0.6 times or less or can particularly preferably suppress to 0.55 times or less.

### (Kinds of miRNA)

Here, about 2600 kinds of small RNA are contained in microparticles derived from a culture supernatant of dental pulp-derived stem cells. About 1800 kinds thereof are miRNAs. Of the miRNAs, miRNAs in high amounts are 180 to 200 kinds. It is characteristic that many of the miRNAs in high amounts in the microparticles derived from dental pulp-derived stem cells are microRNAs related to the treatment of cranial nerve disease or an eye disease, and the characteristic has not been conventionally known but has been newly found by the present inventor. This characteristic is largely different from the kinds of miRNA in high amounts in the microparticles of other mesenchymal stem cells. For example, the miRNAs in high amounts in the microparticles of adipose-derived stem cells and the microparticles of umbilical cord-derived stem cells include almost no microRNAs related to the treatment of cranial nerve disease or an eye disease.

The microparticles derived from a culture supernatant of dental pulp-derived stem cells preferably contain five kinds or more of microRNA which can regulate the expression of a protein related to amyloid β-related or tau protein-related dementia or brain inflammation such as Alzheimer's disease (also referred to as Alzheimer-related microRNAs below), more preferably contain 10 kinds or more, particularly preferably contain 20 kinds or more, further particularly preferably contain 30 kinds or more or most preferably contain 100 kinds or more.

### (Kinds of Microparticles)

The microparticles are preferably of at least one kind selected from the group consisting of exosomes, microvesicles, membrane particles, membrane vesicles, ectosomes, exovesicles and microvesicles, more preferably exosomes.

The diameter of the microparticles is preferably 10 to 1000 nm, more preferably 30 to 500 nm, particularly preferably 50 to 150 nm.

Moreover, molecules called tetraspanins such as CD9, CD63 and CD81 are desirably on the surface of the microparticles, and the molecules may be those of CD9 alone, CD63 alone, CD81 alone or any combination of two or three thereof.

A preferable aspect in which exosomes are used as the microparticles is sometimes explained below, but the microparticles used in the invention are not limited to exosomes.

The exosomes are preferably extracellular vesicles which are released from cells during fusion of plasma membrane and multivesicular bodies.

The surface of the exosomes preferably contains a lipid and a protein derived from the cell membrane of dental pulp-derived stem cells.

The exosomes preferably contain, in their inside, an intracellular substance of dental pulp-derived stem cells such as nucleic acids (microRNAs, messenger RNAs, DNAs and the like) and proteins.

The exosomes are known to be used for communication among cells through transportation of genetic information from a cell to another cell. The exosomes can be tracked easily and can be targeted at a specific region.

### (Microparticle Content)

The microparticle content of the gene expression regulator of the invention is not particularly restricted. The gene expression regulator of the invention preferably contains 0.5×10⁸ particles or more, more preferably 1.0×10⁸ particles or more, particularly preferably 2.0×10⁸ particles or more, further particularly preferably 2.5×10⁸ particles or more, still further particularly preferably 1.0×10⁹ particles or more of the microparticles.

Moreover, the concentration of the microparticles contained in the gene expression regulator of the invention is not particularly restricted. The gene expression regulator of the invention preferably contains the microparticles at 1.0×10⁸ particles/mL or more, more preferably 2.0×10⁸ particles/mL or more, particularly preferably 4.0×10⁸ particles/mL or more, further particularly preferably 5.0×10⁸ particles/mL or more, still further particularly preferably 2.0×10⁹ particles/mL or more.

In a preferable aspect, the gene expression regulator of the invention contains the microparticles in such a high amount or at such a high concentration and thus can maintain the amount of the miRNA or the like high and can regulate (particularly suppress) the expression of a protein related to amyloid β-related or tau protein-related dementia or brain inflammation.

### <Other Components>

The gene expression regulator of the invention may contain another component in addition to the microparticles depending on the species of the animal as the subject of the administration or the purpose as long as the effects of the invention are not impaired. Examples of the other component include nutritional components, antibiotics, cytokines, protectants, carriers, excipients, disintegrating agents, buffers, emulsifiers, suspending agents, soothing agents, stabilizers, preservatives, and antiseptics.

Examples of the nutritional components include fatty acids, and vitamins.

Examples of the antibiotics include penicillin, streptomycin, and gentamicin.

The carriers may be materials known as pharmaceutically acceptable carriers.

The gene expression regulator of the invention may be the culture supernatant itself of dental pulp-derived stem cells or the microparticles themselves or may be a pharmaceutical composition further containing a pharmaceutically acceptable carrier or excipient or the like. The purpose of the pharmaceutical composition is to promote administration of the culture supernatant of dental pulp-derived stem cells or the microparticles to the subject of the administration.

The pharmaceutically acceptable carrier is preferably a carrier (including a diluent) which does not cause significant irritation in the subject of the administration and which does not inhibit the biological activity and the characteristics of the administered composition. Examples of the carrier include propylene glycol, (physiological) saline, an emulsion, a buffer, a medium such as DMEM and RPMI and a low-temperature preservation medium containing a component for removing free radicals.

The gene expression regulator of the invention may contain the active ingredient of a conventionally known gene expression regulator. One skilled in the art can appropriately change depending on the use, the subject of the administration and the like.

The gene expression regulator of the invention preferably does not contain specific substances.

For example, the gene expression regulator of the invention preferably does not contain dental pulp-derived stem cells.

Moreover, the gene expression regulator of the invention preferably does not contain MCP-1. In this regard, however, cytokines other than MCP-1 may be contained. Examples of the other cytokines include those described in [0014] to [0020] of JP2018-023343A.

The gene expression regulator of the invention preferably does not contain Siglec-9. In this regard, however, sialic acid-binding immunoglobulin-like lectins other than Siglec-9 may be contained.

The gene expression regulator of the invention preferably does not substantially contain serum (fetal bovine serum, human serum, sheep serum or the like). Moreover, the gene expression regulator of the invention preferably does not substantially contain the conventional serum replacements such as knockout serum replacement (KSR).

The amounts (solid amounts) of the other components in the gene expression regulator of the invention are each preferably 1 mass% or less, more preferably 0.1 mass% or less, particularly preferably 0.01 mass% or less.

### <Production Method of Gene Expression Regulator>

The method for producing the gene expression regulator of the invention is not particularly restricted.

The gene expression regulator of the invention may be prepared by preparing a culture supernatant of dental pulp-derived stem cells or the like and subsequently purifying microparticles from the culture supernatant of dental pulp-derived stem cells. Alternatively, the gene expression regulator of the invention may be prepared by purifying microparticles from a commercially purchased culture supernatant of dental pulp-derived stem cells. The gene expression regulator of the invention may also be prepared by receiving a composition containing a culture supernatant of dental pulp-derived stem cells which has been disposed of (or by appropriately purifying such a composition) and purifying microparticles from the composition.

### (Preparation Method of Culture Supernatant of Dental Pulp-Derived Stem Cells or the Like)

The culture supernatant of dental pulp-derived stem cells or the like is not particularly restricted.

The culture supernatant of dental pulp-derived stem cells or the like preferably does not substantially contain serum. For example, the serum content of the culture supernatant of dental pulp-derived stem cells or the like is preferably 1 mass% or less, more preferably 0.1 mass% or less, particularly preferably 0.01 mass% or less.

The dental pulp-derived stem cells may be derived from a human or from a nonhuman animal. The nonhuman animal may be the same as the animal (species) as the subject of the administration of the gene expression regulator of the invention described below and is preferably a mammal.

The dental pulp-derived stem cells used for the culture supernatant are not particularly restricted. Dental pulp stem cells from exfoliated deciduous teeth, deciduous dental pulp stem cells obtained by another method and permanent dental pulp stem cells (DPSCs) can be used. In addition to human deciduous dental pulp stem cells and human permanent dental pulp stem cells, dental pulp-derived stem cells derived from a nonhuman animal such as pig deciduous dental pulp stem cells can be used.

The dental pulp-derived stem cells can produce, in addition to exosomes, vascular endothelial growth factors (VEGFs), hepatocyte growth factors (HGFs), insulin-like growth factors (IGFs), platelet-derived growth factors (PDGFs), transforming growth factors-beta (TGF-β)-1 and -3, TGF-α, KGF, HBEGF, SPARC, other growth factors and various cytokines such as chemokine. Many other bioactive substances can also be produced.

In the invention, the dental pulp-derived stem cells used for the culture supernatant of dental pulp-derived stem cells are particularly preferably dental pulp-derived stem cells containing many proteins, and deciduous dental pulp stem cells are preferably used. That is, in the invention, a culture supernatant of deciduous dental pulp stem cells is preferably used.

The dental pulp-derived stem cells used in the invention may be natural cells or genetically modified cells as long as the intended treatment can be achieved.

In particular, in the invention, immortalized stem cells of dental pulp-derived stem cells can be used. Using immortalized stem cells, which are capable of proliferating substantially infinitely, the amounts and the compositions of the biological factors contained in the culture supernatant of the stem cells can be stabilized over a long period of time. The immortalized stem cells of dental pulp-derived stem cells are not particularly restricted. The immortalized stem cells are preferably non-tumor immortalized stem cells. The immortalized stem cells of dental pulp-derived stem cells can be prepared by adding one of the following small molecule compounds (inhibitors) or any combination thereof to dental pulp-derived stem cells and culturing the cells.

TGFβ receptor inhibitors are not particularly limited as long as the TGFβ receptor inhibitors have the action of inhibiting the function of transforming growth factor (TGF) β receptor, and examples thereof include 2-(5-benzo[1,3]dioxol-4-yl-2-tert-butyl-1H-imidozol-4-yl)-6-methylpyridine, 3-(6-methylpyridin-2-yl)-4-(4-quinolyl)-1-phenylthiocarbamoyl-1H-pyrazole (A-83-01), 2-[(5-chloro-2-fluorophenyl)pteridin-4-yl]pyridin-4-ylamine (SD-208), 3-[(pyridin-2-yl)-4-(4-quinonyl)]-1H-pyrazole and 2-(3-(6-methylpyridin-2-yl)-1H-pyrazol-4-yl)-1,5-naphthyridine (all are from Merck), and SB431542 (Sigma-Aldrich). A-83-01 is preferable.

ROCK inhibitors are not particularly limited as long as the ROCK inhibitors have the action of inhibiting the function of Rho-binding kinase. Examples of the ROCK inhibitors include GSK269962A (Axonmedchem), Fasudil hydrochloride (Tocris Bioscience), and Y-27632 and H-1152 (both are from FUJIFILM Wako Pure Chemical Corporation). Y-27632 is preferable.

GSK3 inhibitors are not particularly limited as long as GSK-3 (glycogen synthase kinase 3) is inhibited, and examples thereof include A 1070722, BIO and BIO-acetoxime (all are from TOCRIS).

MEK inhibitors are not particularly limited as long as the MEK inhibitors have the action of inhibiting the function of MEK (MAP kinase-ERK kinase), and examples thereof include AZD6244, CI-1040 (PD184352), PD0325901, RDEA119 (BAY86-9766), SL327 and U0126-EtOH (all are from Selleck), and PD98059, U0124 and U0125 (all are from Cosmo Bio Co., Ltd.).

When the gene expression regulator of the invention is used for regenerative medicine, due to the requirements of the Act on the Safety of Regenerative Medicine, the culture supernatant of dental pulp-derived stem cells or immortalized stem cells thereof or a composition containing microparticles derived from the cells does not contain other somatic stem cells than the dental pulp-derived stem cells or the like. The gene expression regulator of the invention may contain mesenchymal stem cells other than the dental pulp-derived stem cells or the like or other somatic stem cells but preferably does not contain such cells.

Examples of the somatic stem cells other than the mesenchymal stem cells include stem cells derived from the dermal system, the digestive system, the myeloid system, and the nerve system, but the somatic stem cells are not limited to the examples. Examples of the somatic stem cells of the dermal system include epithelial stem cells, and hair follicle stem cells. Examples of the somatic stem cells of the digestive system include pancreatic (general) stem cells, and hepatic stem cells. Examples of the somatic stem cells of the myeloid system (except for the mesenchymal stem cells) include hematopoietic stem cells. Examples of the somatic stem cells of the nerve system include neural stem cells, and retinal stem cells.

The gene expression regulator of the invention may contain stem cells other than somatic stem cells but preferably does not contain such stem cells. The stem cells other than somatic stem cells include embryonic stem cells (ES cells), induced pluripotent stem cells (iPS cells) and embryonal carcinoma cells (EC cells).

The method for preparing the culture supernatant of dental pulp-derived stem cells or immortalized stem cells thereof is not particularly restricted, and a conventional method can be used.

The culture supernatant of dental pulp-derived stem cells or the like is a culture solution obtained by culturing dental pulp-derived stem cells. For example, a culture supernatant which can be used for the invention can be obtained by culturing dental pulp-derived stem cells and then separating and removing the cell components. A culture supernatant which has been appropriately subjected to various treatments (for example, centrifugation, concentration, solvent substitution, dialysis, freezing, drying, lyophilization, dilution, desalination, preservation or the like) may also be used.

The dental pulp-derived stem cells for obtaining the culture supernatant of dental pulp-derived stem cells can be selected by a normal method and can be selected based on the size and the morphology of the cells or as adherent cells. The cells can be selected from dental pulp cells collected from an exfoliated deciduous tooth or a permanent tooth as adherent cells or passaged cells thereof. The culture supernatant of dental pulp-derived stem cells used can be a culture supernatant obtained by culturing selected stem cells.

The "culture supernatant of dental pulp-derived stem cells or the like" is preferably a culture solution which does not contain the cells themselves obtained by culturing the dental pulp-derived stem cells or the like. The culture supernatant of dental pulp-derived stem cells used in the invention preferably does not contain any cells (regardless of the kinds of cells) as a whole in an aspect. Due to the feature, the composition of the aspect is clearly distinguished of course from the dental pulp-derived stem cells themselves and also from various compositions containing dental pulp-derived stem cells. A typical example of the aspect is a composition which does not contain dental pulp-derived stem cells and which is composed only of a culture supernatant of dental pulp-derived stem cells.

The culture supernatant of dental pulp-derived stem cells used in the invention may contain a culture supernatant of both deciduous dental pulp-derived stem cells and adult dental pulp-derived stem cells. The culture supernatant of dental pulp-derived stem cells used in the invention preferably contains a culture supernatant of deciduous dental pulp-derived stem cells as an active ingredient, more preferably in an amount of 50 mass% or more, preferably 90 mass% or more. The culture supernatant of dental pulp-derived stem cells used in the invention is further particularly preferably a composition composed only of a culture supernatant of deciduous dental pulp-derived stem cells.

A basal medium, a basal medium supplemented with serum and the like or the like can be used for the culture solution of dental pulp-derived stem cells for obtaining the culture supernatant. Examples of the basal medium which can be used include, in addition to Dulbecco's modified Eagle's medium (DMEM), Iscove's modified Dulbecco's medium (IMDM) (GIBCO and the like), Ham's F12 medium (SIGMA, GIBCO and the like), and RPMI1640 medium. Examples of the component that can be added to the medium include serum (fetal bovine serum, human serum, sheep serum and the like), serum replacements (knockout serum replacement (KSR) and the like), bovine serum albumin (BSA), antibiotics, vitamins and minerals.

In order to prepare "a culture supernatant of dental pulp-derived stem cells" which does not contain serum, however, a serum-free medium should be used throughout the whole process or for the last passaging culture or the last passaging culture steps. For example, when dental pulp-derived stem cells are cultured in a medium which does not contain serum (a serum-free medium), a culture supernatant of dental pulp-derived stem cells which does not contain serum can be prepared. When passaging culture is conducted once or two or more times and when the last passaging culture or the last passaging culture steps are conducted in a serum-free medium, a culture supernatant of dental pulp-derived stem cells or the like which does not contain serum can also be obtained. Moreover, a culture supernatant of dental pulp-derived stem cells which does not contain serum can also be obtained by removing serum from the recovered culture supernatant using dialysis, solvent substitution using a column or the like.

For culturing the dental pulp-derived stem cells for obtaining the culture supernatant, generally used conditions can be applied as they are. The method for preparing a culture supernatant of the dental pulp-derived stem cells can be the same as the cell culture method described below except that the isolation and selection steps of the stem cells are appropriately adjusted to the type of the stem cells. One skilled in the art can appropriately isolate and select dental pulp-derived stem cells according to the type of the dental pulp-derived stem cells.

In order to produce a large amount of microparticles such as exosomes, special conditions may be applied for culturing the dental pulp-derived stem cells. The special conditions may be, for example, low-temperature conditions, low-oxygen conditions, microgravity conditions, conditions of coculturing with any stimulant or the like.

The culture supernatant of dental pulp-derived stem cells used for preparing the microparticles such as exosomes in the invention may contain another component in addition to the culture supernatant of dental pulp-derived stem cells but preferably does not substantially contain any other component.

The culture supernatant of dental pulp-derived stem cells may be stored after an additive used for preparing exosomes is added to the culture supernatant.

### (Preparation Method of Microparticles)

Microparticles can be prepared by purifying the microparticles from the culture supernatant of dental pulp-derived stem cells or the like.

The purification of the microparticles is preferably separation of a fraction containing the microparticles from the culture supernatant of dental pulp-derived stem cells, more preferably isolation of the microparticles.

The microparticles can be isolated by separation from non-associated components based on the characteristics of the microparticles. For example, the microparticles can be isolated based on the molecular weight, the size, the morphology, the composition or the biological activity.

In the invention, the microparticles can be purified by centrifuging the culture supernatant of dental pulp-derived stem cells and collecting a specific fraction containing a large amount of the microparticles (for example, precipitates) obtained by the centrifugation. Unnecessary components (insoluble components) in a fraction other than the predetermined fraction may be removed. The solvent, the dispersion medium and the unnecessary components do not have to be removed completely from the gene expression regulator of the invention. An example of the centrifugation conditions is 100 to 20000 g for 1 to 30 minutes.

In the invention, the microparticles can be purified by subjecting the culture supernatant of dental pulp-derived stem cells or a centrifuged product thereof to filtration. Unnecessary components can be removed through the filtration. Moreover, when a filter membrane having an appropriate pore size is used, the removal of the unnecessary components and sterilization can be conducted simultaneously. The material, the pore size and the like of the filter membrane used for the filtration are not particularly limited. The filtration can be achieved by a known method using a filter membrane having an appropriate molecular weight or an appropriate size cut-off. Because exosomes can be easily fractionated, the pore size of the filter membrane is preferably 10 to 1000 nm, more preferably 30 to 500 nm, particularly preferably 50 to 150 nm.

In the invention, the culture supernatant of dental pulp-derived stem cells, a centrifuged product thereof or a filtered product thereof can be separated further using separation means such as RAM chromatography. For example, high-performance liquid chromatography (HPLC) using any of various columns can be used. The column which can be used is a size exclusion column or a binding column.

In order to track the microparticles (or the activity thereof) in the fraction at each treatment stage, one or more characteristics or the biological activity of the microparticles can be used. For example, in order to track the microparticles, light scattering, refractive index, dynamic light scattering or UV-VIS detector can be used. Alternatively, in order to track the activity in each fraction, specific enzymatic activity or the like can be used.

As the method for purifying the microparticles, the method described in [0034] to [0064] of JP2019-524824A may be used, and the contents of the publication are incorporated in the present description by reference.

The final form of the gene expression regulator of the invention is not particularly restricted. For example, the gene expression regulator of the invention may be in a form in which the microparticles are packed in a container with a solvent or a dispersion medium, a form in which the microparticles are formed into gel with gel and packed in a container, a form in which the microparticles are solidified by freezing and/or drying and formulated or packed in a container or another form. Examples of the container include a tube, a centrifuge tube, or a bag which is suitable for cryopreservation. The freezing temperature can be, for example, -20°C to -196°C.

Compared to the conventional compositions which can be used as gene expression regulators, the gene expression regulator of the invention has advantages such as easiness of mass production, utilization of culture solutions of stem cells which are otherwise disposed of as industrial waste or the like and decreased cost of the disposal of culture solutions of stem cells. In particular, when the culture supernatant of dental pulp-derived stem cells is a culture supernatant of human dental pulp-derived stem cells, the gene expression regulator of the invention also has advantages of high safety for example from the immunological point of view and less ethical issues in application to a human. When the culture supernatant of dental pulp-derived stem cells is a culture supernatant of dental pulp-derived stem cells of a patient with dementia, the application of the gene expression regulator of the invention to such a patient will be safer and cause less ethical issues.

When the gene expression regulator of the invention is derived from a culture supernatant of dental pulp-derived stem cells, the gene expression regulator is used also for applications in restorative medicine. In particular, a composition containing microparticles derived from a culture supernatant of dental pulp-derived stem cells or the like is preferably used for applications in restorative medicine. Here, in regenerative medicine based on stem cell transplantation, it is known that stem cells do not play the main role in regeneration and that liquid components produced by the stem cells repair the organ with the autologous stem cells. Difficult problems of the conventional stem cell transplantation, such as malignant transformation, standardization, the administration method, preservation and the culture method, are solved, and restorative medicine is enabled by the culture supernatant of dental pulp-derived stem cells or a composition using microparticles derived therefrom. Compared to stem cell transplantation, the case using the gene expression regulator of the invention does not easily cause neoplastic transformation or the like and is considered to be safer because cell transplantation is not conducted. Moreover, the gene expression regulator of the invention has advantages because those with uniformly standardized quality can be used. Because mass production and an efficient administration method can be selected, use with a low cost is enabled.

### [Improvement Method of Dementia]

The method for improving dementia of the invention includes administering an effective amount of the gene expression regulator of the invention to a subject which has developed dementia.

The step of administering the gene expression regulator of the invention to a subject which has developed dementia is not particularly restricted.

The administration method can be spraying or inhalation to the oral cavity, the nasal cavity or the respiratory tract, infusion, local administration, nasal drops or the like and is preferably little invasive. The local administration method is preferably an injection. Another preferable method is electroporation, in which voltage (electric pulses) is applied to the skin surface to create temporal fine holes in the cell membrane, allowing the active ingredient to penetrate to the dermis layer, which cannot be reached by normal care. The local administration may be intravenous administration, intraarterial administration, intraportal administration, intradermal administration, subcutaneous administration, intramuscular administration, intraperitoneal administration or the like and is more preferably intraarterial administration, intravenous administration, subcutaneous administration or intraperitoneal administration.

Moreover, using various formulation techniques, the *in vivo* distribution of the gene expression regulator can be changed. Many methods for changing the *in vivo* distribution are known to one skilled in the art. An example of such a method is, for example, protection of exosomes in vesicles composed of a substance such as protein, a lipid (for example, liposome), a carbohydrate and a synthetic polymer.

The gene expression regulator of the invention which has been administered to a subject which has developed dementia circulates in the body of the subject and may reach a certain tissue.

The number of administrations and the intervals of administration are not particularly restricted. The number of administrations can be once a week or more and is preferably five times or more, more preferably six times or more, particularly preferably seven times or more. The intervals of administration are preferably an hour to a week, more preferably half a day to a week, particularly preferably a day (once a day). However, the number and the intervals can be appropriately adjusted in accordance with the species of the subject of the administration and the symptom of the subject of the administration.

The gene expression regulator of the invention is preferably used for administration of the gene expression regulator to a subject which has developed dementia once a week or more for a therapeutically effective period. When the subject of the administration is a human, the number of administrations per week is preferably higher, and the gene expression regulator is preferably administered five times a week or more for a therapeutically effective period or preferably administered every day.

When a culture supernatant of dental pulp-derived stem cells of a concentration of 2.0×10⁹ cells/ml is used, 0.1 to 5 ml per mouse (about 25 g) is preferable in a mouse model, 0.3 to 3 ml is more preferable, and 0.5 to 1 ml is further particularly preferable.

When microparticles of a concentration of 0.1×10⁸ particles/µg are used, 1 to 50 µg per mouse (about 25 g) is preferable in a mouse model, 3 to 30 µg is more preferable, and 5 to 25 µg is further particularly preferable.

A preferable range of the dose per body weight for another animal can be calculated from the dose per body weight (about 25 g) of a model mouse using the proportionality relation. The range, however, can be appropriately adjusted depending on the symptom of the subject of the administration.

The animal (species) as the subject to which the gene expression regulator of the invention is administered is not particularly restricted. The animal as the subject to which the gene expression regulator of the invention is administered is preferably a mammal, a bird (chicken, quail, duck or the like) or fish (salmon, trout, tuna, bonito or the like). The mammal may be a human or a nonhuman mammal but is particularly preferably a human. The nonhuman mammal is more preferably a cow, a pig, a horse, a goat, a sheep, a monkey, a dog, a cat, a mouse, a rat, a guinea pig or a hamster.

The gene expression regulator of the invention may be used in combination with a conventionally known gene expression regulator. Moreover, the gene expression regulator of the invention may be used in combination with a conventionally known agent for treating or preventing amyloid β-related or tau protein-related dementia or brain inflammation.

### Examples

The characteristics of the invention are explained further specifically referring to Examples and Comparative Examples or Reference Examples below. The materials, the amounts, the proportions, the contents of treatments, the procedures of treatments and the like shown in the Examples below can be appropriately changed as long as such changes do not go beyond the intention of the invention. Accordingly, the scope of the invention should not be construed as being limited by the specific examples shown below.

### [Example 1]

### <Preparation of Culture Supernatant of Dental Pulp-Derived Stem Cells>

A culture supernatant of human deciduous dental pulp stem cells was prepared, and the culture supernatant was fractionated, according to the method described in Example 6 of JP6296622B except that DMEM medium was used instead of the DMEM/HamF12 mixture medium. For the primary culture, fetal bovine serum (FBS) was added to the culture. In the passaging culture, the supernatant was taken from the passaged culture solution which was cultured using the primary culture solution in such a manner that FBS would not be contained, and a culture supernatant of deciduous dental pulp stem cells was thus prepared. Here, DMEM is Dulbecco's modified Eagle's medium, and F12 is Ham's F12 medium.

### <Preparation of Exosomes>

Exosomes of the dental pulp-derived stem cells were purified and recovered from the obtained culture supernatant of dental pulp-derived stem cells by the following ultracentrifugation method.

After filtering the culture supernatant of deciduous dental pulp stem cells (100 mL) with a filter having a pore size of 0.22 micrometers, the solution was centrifuged for 60 minutes at 4°C at 100000× g. The supernatant was decanted, and the exosome-concentrated pellets were re-suspended in phosphate-buffered saline (PBS). The re-suspended sample was centrifuged for 60 minutes at 100000× g. The pellets were again recovered from the bottom of the centrifuge tube as the concentrated sample (about 100 µl). The protein concentration was determined using micro BSA protein assay kit (Pierce, Rockford, IL). The composition containing exosomes (concentrated solution) was stored at -80°C.

The composition containing the exosomes purified from the culture supernatant of dental pulp-derived stem cells was used as a gene expression regulator sample of Example 1.

The average particle size and the concentration of the microparticles contained in the gene expression regulator of Example 1 were evaluated using nanoparticle analysis system NanoSight (manufactured by Quantum Design Japan, Inc.).

The average particle size of the microparticles contained in the gene expression regulator of Example 1 was 50 to 150 nm.

The gene expression regulator of Example 1 was an exosome solution having a high concentration of 1.0×10⁹ particles/ml, specifically an exosome solution having a high concentration of 2.0×10⁹ particles/ml.

Moreover, the components of the obtained gene expression regulator of Example 1 were analyzed by a known method. As a result, it was found that the gene expression regulator of Example 1 did not contain any stem cells of dental pulp-derived stem cells, did not contain MCP-1 and did not contain Siglec-9. It was thus found that an active ingredient which is different from MCP-1 and Siglec-9, which are active ingredients of a culture supernatant of mesenchymal stem cells, and from analogs thereof is the active ingredient of the gene expression regulator of Example 1.

### [Comparative Example 1]

### <Preparation of Culture Supernatant of Adipose-Derived Stem Cells>

A culture supernatant of adipose-derived stem cells was prepared in the same manner as in Example 1 except for using adipose-derived stem cells.

Exosomes of adipose-derived stem cells were purified in the same manner as in Example 1 except for using the culture supernatant of adipose-derived stem cells. The obtained exosomes of adipose-derived stem cells were used as the gene expression suppressor of Comparative Example 1.

### [Comparative Example 2]

### <Preparation of Culture Supernatant of Umbilical Cord-Derived Stem Cells>

A culture supernatant of umbilical cord-derived stem cells was prepared in the same manner as in Example 1 except for using umbilical cord-derived stem cells.

Exosomes of umbilical cord-derived stem cells were purified in the same manner as in Example 1 except for using the culture supernatant of umbilical cord-derived stem cells. The obtained exosomes of umbilical cord-derived stem cells were used as the gene expression suppressor of Comparative Example 2.

### [Test Example 1]: microRNAs Expressed in Exosomes

The small RNAs contained in the gene expression suppressor of Example 1 were analyzed by next-generation sequencing (NGS) analysis. By the NGS analysis, 1787 miRNAs contained in the gene expression suppressor of Example 1 (the exosomes of dental pulp-derived stem cells) were identified. The obtained results are shown in Table 2 below.

**[Table 2]**

| name | Detected Number | % |
|---|---|---|
| miRNA | 1787 | 72.35 |
| piRNA | 123 | 4.98 |
| tRNA | 411 | 16.64 |
| other RNA | 149 | 6.03 |
| Total | 2470 | 100 |

### [Test Example 2]: Comparison with miRNAs in Cerebral Cortex

IMOTA (Interactive Multi-Omics-Tissue Atlas) was used for analyzing the microRNAs.

MicroRNAs regulating proteins were searched with IMOTA mainly in the cerebral cortex. IMOTA is interactive multi omics atlas which can examine the interactions and the expression levels of miRNAs, mRNAs and proteins in tissues and cells (Nucleic Acids Research, Volume 46, Issue D1, 4 January 2018, Pages D770-D775, "IMOTA: an interactive multi-omics tissue atlas for the analysis of human miRNA-target interactions"). In the cerebral cortex, the Omic protein count was 3071, the mRNA count was 14182, and the miRNA count was 1124. Moreover, the Omic count of the proteins with a high expression rate in the cerebral cortex was 1119, the mRNA count was 1110, and the miRNA count was 145.

There were 859 miRNAs which were commonly expressed among the 1124 miRNAs expressed in the cerebral cortex and the 1787 miRNAs expressed in the exosomes purified from the culture supernatant of dental pulp-derived stem cells (the gene expression regulator of Example 1; SGF exosome). This means that the proportion of the miRNAs which were expressed also in the cerebral cortex, of the miRNAs expressed in the gene expression suppressor of Example 1 (the exosomes of dental pulp-derived stem cells), was 48.1%, which was extremely high.

Moreover, the miRNAs which were expressed in the gene expression suppressor of Example 1 (the exosomes of dental pulp-derived stem cells) and in which the expression in the cerebral cortex was high were the 122 miRNAs shown in Table 3 and Table 4 below.

**[Table 3]**

| | | | |
|---|---|---|---|
| hsa-let-7a-5p | hsa-miR-125b-5p | hsa-miR-204-5p | hsa-miR-30d-5p |
| hsa-let-7b-5p | hsa-miR-126-3p | hsa-miR-21-5p | hsa-miR-30e-5p |
| hsa-let-7c-5p | hsa-miR-1260a | hsa-miR-211-3p | hsa-miR-3135b |
| hsa-let-7d-5p | hsa-miR-1268a | hsa-miR-218-5p | hsa-miR-3141 |
| hsa-let-7e-5p | hsa-miR-1268b | hsa-miR-219a-5p | hsa-miR-3162-5p |
| hsa-let-7f-5p | hsa-miR-1273g-3p | hsa-miR-22-3p | hsa-miR-3196 |
| hsa-let-7g-5p | hsa-miR-128-3p | hsa-miR-221-3p | hsa-mi R-320c |
| hsa-let-7i-5p | hsa-miR-129-2-3p | hsa-miR-23a-3p | hsa-miR-331-3p |
| hsa-miR-100-5p | hsa-miR-130a-3p | hsa-miR-23b-3p | hsa-miR-338-3p |
| hsa-miR-101-3p | hsa-miR-136-5p | hsa-miR-24-3p | hsa-miR-342-3p |
| hsa-miR-103a-3p | hsa-miR-140-5p | hsa-miR-26a-5p | hsa-miR-34a-5p |
| hsa-miR-107 | hsa-miR-142-3p | hsa-miR-26b-5p | hsa-miR-34b-5p |
| hsa-miR-1202 | hsa-miR-144-3p | hsa-miR-27b-3p | hsa-miR-3656 |
| hsa-miR-1207-5p | hsa-miR-15a-5p | hsa-miR-2861 | hsa-miR-3665 |
| hsa-miR-1225-5p | hsa-miR-16-5p | hsa-miR-29a-3p | hsa-miR-3679-5p |
| hsa-miR-1227-5p | hsa-miR-181a-5p | hsa-miR-29b-3p | hsa-miR-376c-3p |
| hsa-miR-1229-5p | hsa-miR-188-5p | hsa-miR-29c-3p | hsa-miR-377-3p |
| hsa-miR-124-3p | hsa-miR-1915-3p | hsa-miR-30a-5p | hsa-miR-3960 |
| hsa-miR-1246 | hsa-miR-195-5p | hsa-mi R-30b-5p | hsa-miR-4270 |
| hsa-miR-125a-3p | hsa-miR-19b-3p | hsa-miR-30c-5p | hsa-miR-4281 |

**[Table 4]**

| | | |
|---|---|---|
| hsa-miR-4284 | hsa-miR-497-5p | hsa-miR-940 |
| hsa-miR-4286 | hsa-miR-5001-5p | hsa-miR-99a-5p |
| hsa-miR-4324 | hsa-miR-5100 | |
| hsa-miR-4442 | hsa-miR-572 | |
| hsa-miR-4443 | hsa-miR-5739 | |
| hsa-miR-4459 | hsa-miR-574-5p | |
| hsa-miR-4466 | hsa-miR-5787 | |
| hsa-miR-4507 | hsa-miR-6068 | |
| hsa-miR-4516 | hsa-miR-6085 | |
| hsa-miR-451a | hsa-miR-6087 | |
| hsa-miR-4530 | hsa-miR-6088 | |
| hsa-miR-4634 | hsa-miR-6089 | |
| hsa-miR-4669 | hsa-miR-6124 | |
| hsa-miR-4739 | hsa-miR-6125 | |
| hsa-miR-4741 | hsa-miR-638 | |
| hsa-miR-4763-3p | hsa-miR-6724-5p | |
| hsa-miR-4800-5p | hsa-miR-7-5p | |
| hsa-miR-483-5p | hsa-miR-9-3p | |
| hsa-miR-488-3p | hsa-miR-9-5p | |
| hsa-miR-494-3p | hsa-miR-939-5p | |

### [Test Example 3]: Search of microRNAs Involved in Disease

Based on the obtained analysis results, microRNAs involved in a disease were searched. miRNAs related to a disease were extracted using IMOTA. Here, whether microRNA which suppresses a protein related to Alzheimer's disease was included or whether microRNA which regulates a target protein of a therapeutic agent was included was examined. In this Test Example 3, microRNAs which suppress β secretase (BACE1) and APP as proteins involved in Alzheimer's disease were searched. MicroRNAs which suppress NMDA, inhibit GSK-3β or suppress PQBP1 as proteins as targets of a therapeutic agent were searched.

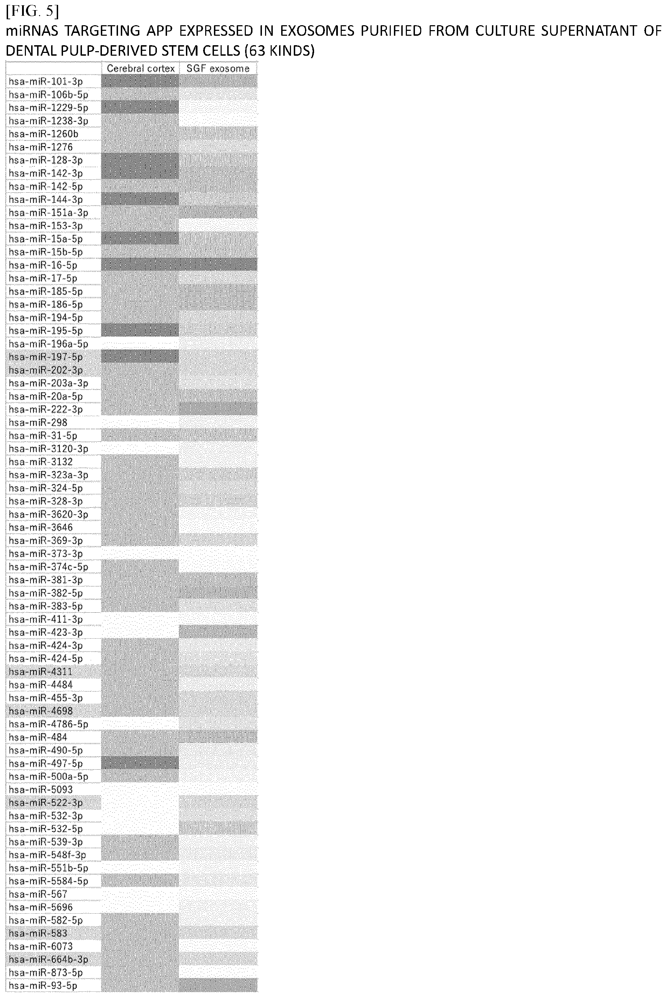

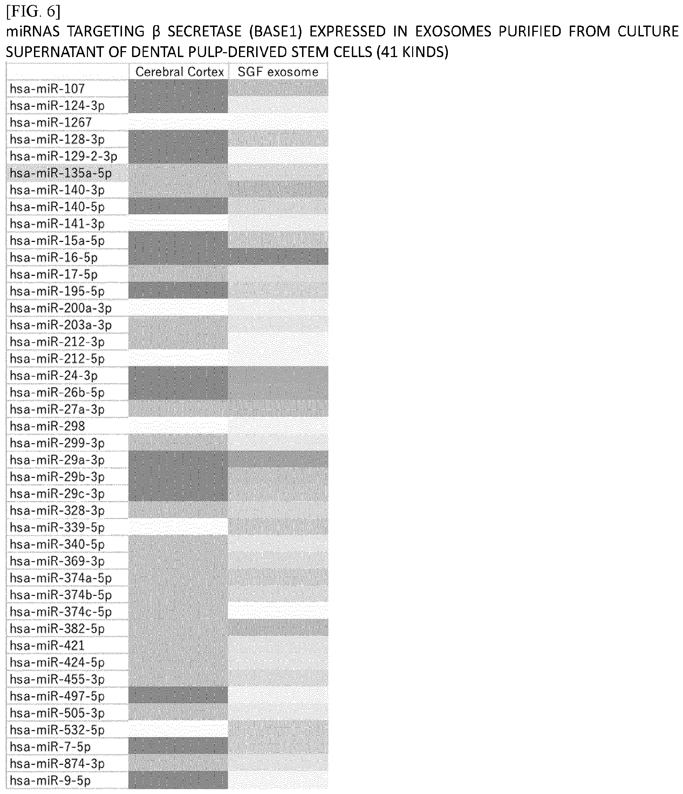

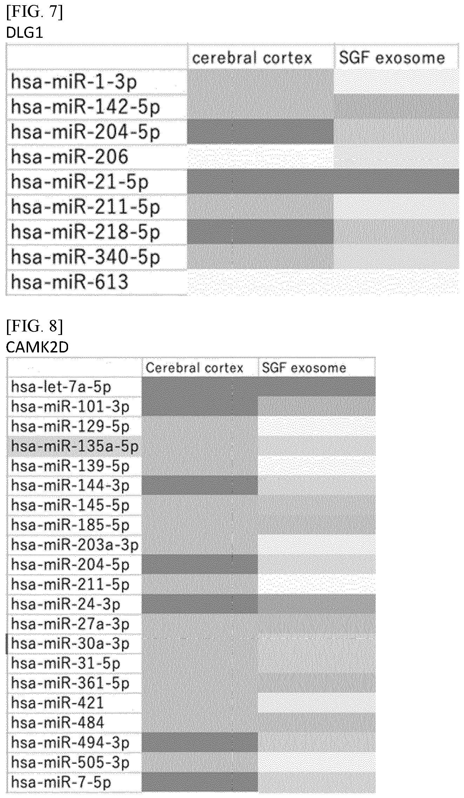

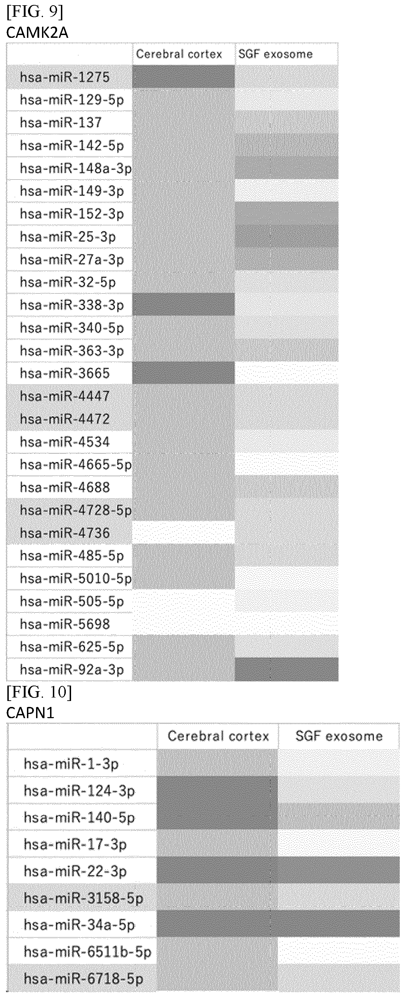

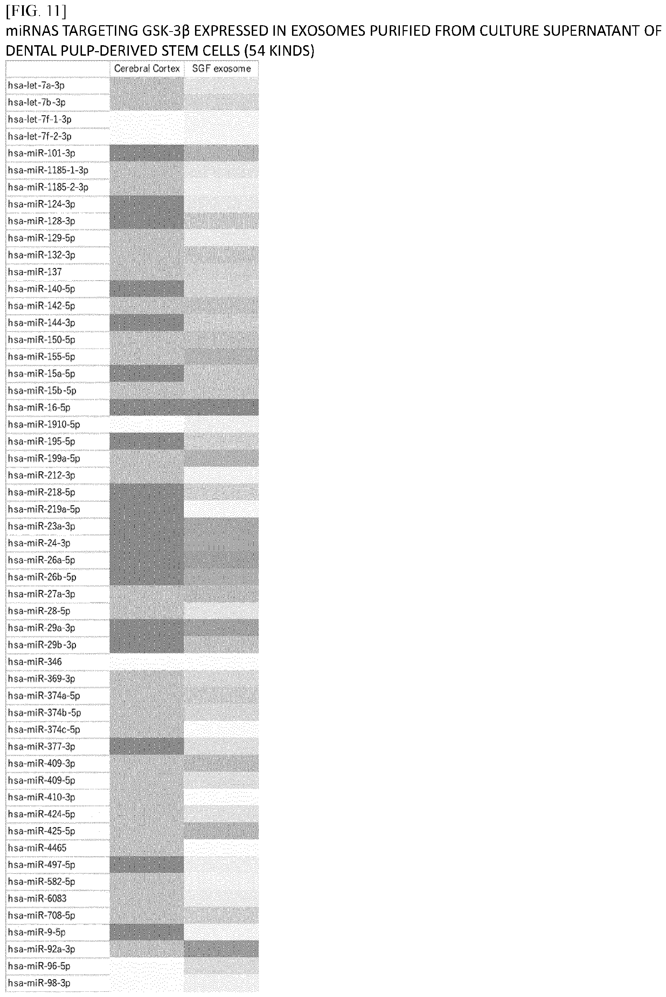

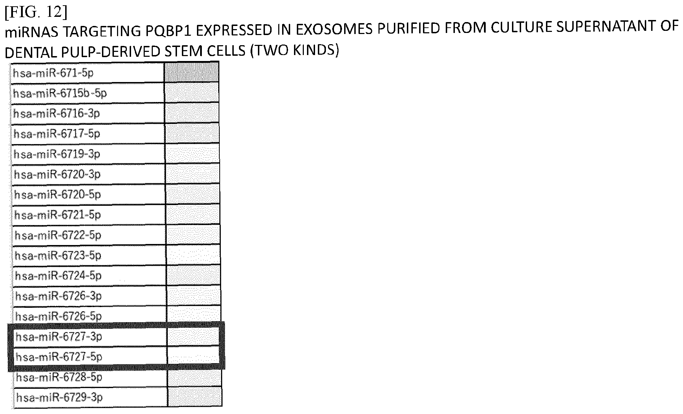

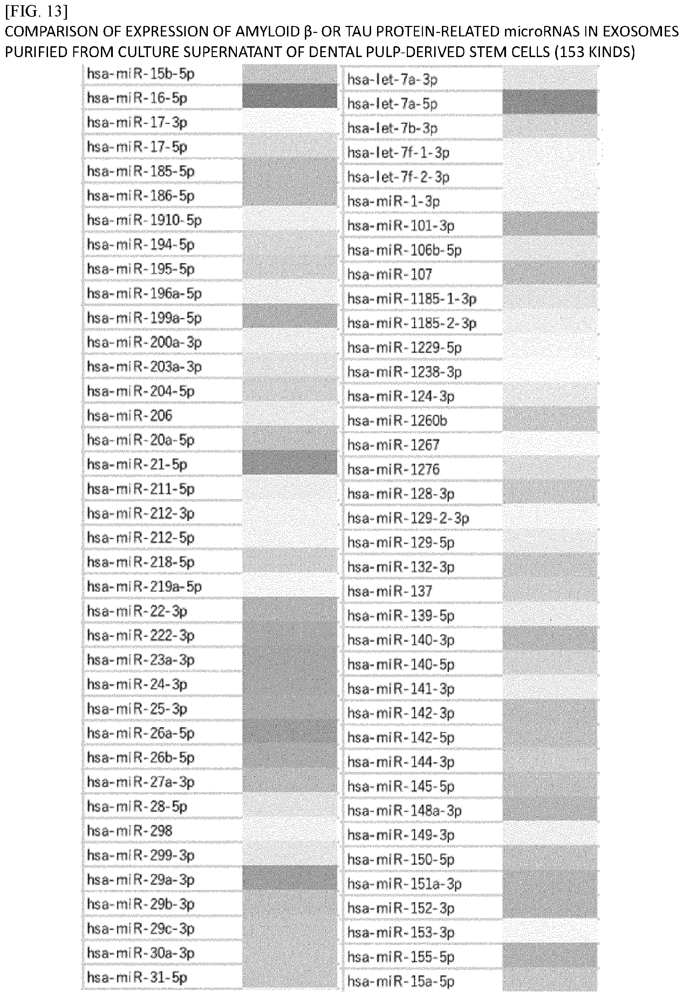

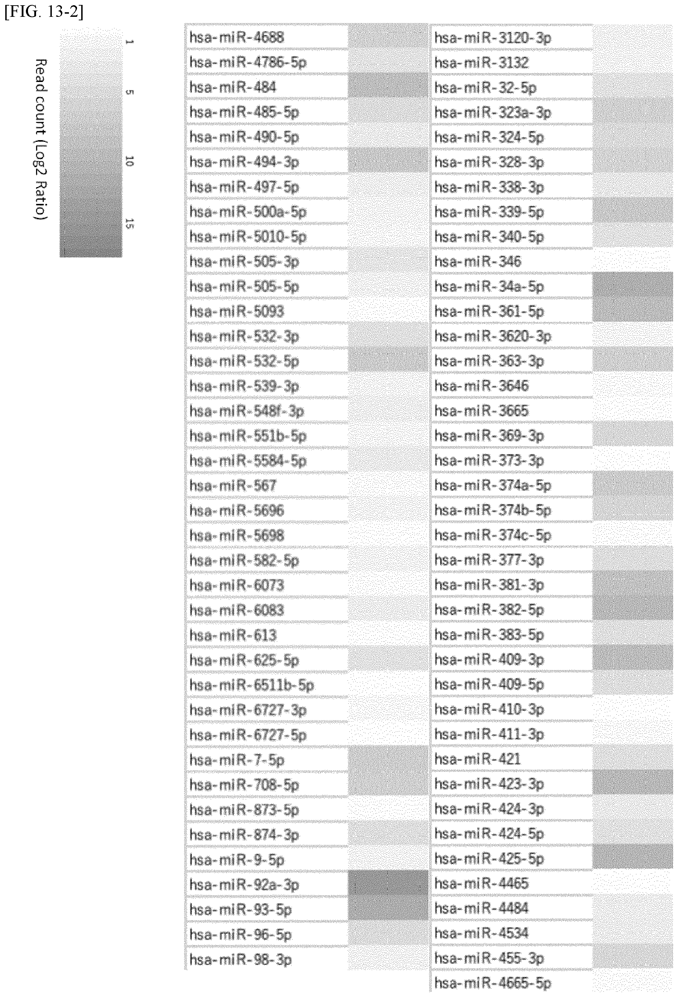

The obtained results are shown in FIG. 5 to FIG. 13-2 in which the expression levels of the miRNAs are compared between the cerebral cortex and the gene expression regulator of Example 1 (SGF).

The miRNAs targeting APP expressed in the exosomes purified from the culture supernatant of dental pulp-derived stem cells were the APP suppression-related miRNA group (63 kinds) below.
hsa-miR-101-3p, hsa-miR-106b-5p, hsa-miR-1229-5p, hsa-miR-1238-3p, hsa-miR-1260b, hsa-miR-1276, hsa-miR-128-3p, hsa-miR-142-3p, hsa-miR-142-5p, hsa-miR-144-3p, hsa-miR-151a-3p, hsa-miR-153-3p, hsa-miR-15a-5p, hsa-miR-15b-5p, hsa-miR-16-5p, hsa-miR-17-5p, hsa-miR-185-5p, hsa-miR-186-5p, hsa-miR-194-5p, hsa-miR-195-5p, hsa-miR-196a-5p, hsa-miR-203a-3p, hsa-miR-20a-5p, hsa-miR-222-3p, hsa-miR-298, hsa-miR-31-5p, hsa-miR-3120-3p, hsa-miR-3132, hsa-miR-323a-3p, hsa-miR-324-5p, hsa-miR-328-3p, hsa-miR-3620-3p, hsa-miR-3646, hsa-miR-369-3p, hsa-miR-373-3p, hsa-miR-374c-5p, hsa-miR-381-3p, hsa-miR-382-5p, hsa-miR-383-5p, hsa-miR-411-3p, hsa-miR-423-3p, hsa-miR-424-3p, hsa-miR-424-5p, hsa-miR-4484, hsa-miR-455-3p, hsa-miR-4786-5p, hsa-miR-484, hsa-miR-490-5p, hsa-miR-497-5p, hsa-miR-500a-5p, hsa-miR-5093, hsa-miR-532-3p, hsa-miR-532-5p, hsa-miR-539-3p, hsa-miR-548f-3p, hsa-miR-551b-5p, hsa-miR-5584-5p, hsa-miR-567, hsa-miR-5696, hsa-miR-582-5p, hsa-miR-6073, hsa-miR-873-5p and hsa-miR-93-5p.

The miRNAs targeting β secretase (BASE1) expressed in the exosomes purified from the culture supernatant of dental pulp-derived stem cells were the BACE1 inhibition-related miRNA group (41 kinds) below.
hsa-miR-107, hsa-miR-124-3p, hsa-miR-1267, hsa-miR-128-3p, hsa-miR-129-2-3p, hsa-miR-140-3p, hsa-miR-140-5p, hsa-miR-141-3p, hsa-miR-15a-5p, hsa-miR-16-5p, hsa-miR-17-5p, hsa-miR-195-5p, hsa-miR-200a-3p, hsa-miR-203a-3p, hsa-miR-212-3p, hsa-miR-212-5p, hsa-miR-24-3p, hsa-miR-26b-5p, hsa-miR-27a-3p, hsa-miR-298, hsa-miR-299-3p, hsa-miR-29a-3p, hsa-miR-29b-3p, hsa-miR-29c-3p, hsa-miR-328-3p, hsa-miR-339-5p, hsa-miR-340-5p, hsa-miR-369-3p, hsa-miR-374a-5p, hsa-miR-374b-5p, hsa-miR-374c-5p, hsa-miR-382-5p, hsa-miR-421, hsa-miR-424-5p, hsa-miR-455-3p, hsa-miR-497-5p, hsa-miR-505-3p, hsa-miR-532-5p, hsa-miR-7-5p, hsa-miR-874-3p and hsa-miR-9-5p.

The miRNAs targeting a protein involved in the activation of NMDA expressed in the exosomes purified from the culture supernatant of dental pulp-derived stem cells were the DLG1 suppression-related miRNA group below, the CAMK2D suppression-related miRNA group below, the CAMK2A suppression-related miRNA group below and the CAPN1 suppression-related miRNA group below (51 kinds in total).

The DLG1 suppression-related miRNA group:
hsa-miR-1-3p, hsa-miR-142-5p, hsa-miR-204-5p, hsa-miR-206, hsa-miR-21-5p, hsa-miR-218-5p, hsa-miR-340-5p and hsa-miR-613.

The CAMK2D suppression-related miRNA group:
hsa-let-7a-5p, hsa-miR-101-3p, hsa-miR-129-5p, hsa-miR-139-5p, hsa-miR-144-3p, hsa-miR-145-5p, hsa-miR-185-5p, hsa-miR-203a-3p, hsa-miR-204-5p, hsa-miR-211-5p, hsa-miR-24-3p, hsa-miR-27a-3p, hsa-miR-30a-3p, hsa-miR-31-5p, hsa-miR-361-5p, hsa-miR-421, hsa-miR-484, hsa-miR-494-3p, hsa-miR-505-3p and hsa-miR-7-5p.

The CAMK2A suppression-related miRNA group:
hsa-miR-129-5p, hsa-miR-137, hsa-miR-142-5p, hsa-miR-148a-3p, hsa-miR-149-3p, hsa-miR-152-3p, hsa-miR-25-3p, hsa-miR-27a-3p, hsa-miR-32-5p, hsa-miR-338-3p, hsa-miR-340-5p, hsa-miR-363-3p, hsa-miR-3665, hsa-miR-4534, hsa-miR-4665-5p, hsa-miR-4688, hsa-miR-485-5p, hsa-miR-5010-5p, hsa-miR-505-5p, hsa-miR-5698, hsa-miR-625-5p and hsa-miR-92a-3p.

The CAPN1 suppression-related miRNA group:
hsa-miR-1-3p, hsa-miR-124-3p, hsa-miR-140-5p, hsa-miR-17-3p, hsa-miR-22-3p, hsa-miR-34a-5p and hsa-miR-6511b-Sp.

### (Total 51 kinds)

The miRNAs targeting GSK-3β expressed in the exosomes purified from the culture supernatant of dental pulp-derived stem cells were the GSK-3β inhibition-related miRNA group (54 kinds) below.
hsa-let-7a-3p, hsa-let-7b-3p, hsa-let-7f-1-3p, hsa-let-7f-2-3p, hsa-miR-101-3p, hsa-miR-1185-1-3p, hsa-miR-1185-2-3p, hsa-miR-124-3p, hsa-miR-128-3p, hsa-miR-129-5p, hsa-miR-132-3p, hsa-miR-137, hsa-miR-140-5p, hsa-miR-142-5p, hsa-miR-144-3p, hsa-miR-150-5p, hsa-miR-155-5p, hsa-miR-15a-5p, hsa-miR-15b-5p, hsa-miR-16-5p, hsa-miR-1910-5p, hsa-miR-195-5p, hsa-miR-199a-5p, hsa-miR-212-3p, hsa-miR-218-5p, hsa-miR-219a-5p, hsa-miR-23a-3p, hsa-miR-24-3p, hsa-miR-26a-5p, hsa-miR-26b-5p, hsa-miR-27a-3p, hsa-miR-28-5p, hsa-miR-29a-3p, hsa-miR-29b-3p, hsa-miR-346, hsa-miR-369-3p, hsa-miR-374a-5p, hsa-miR-374b-5p, hsa-miR-374c-5p, hsa-miR-377-3p, hsa-miR-409-3p, hsa-miR-409-5p, hsa-miR-410-3p, hsa-miR-424-5p, hsa-miR-425-5p, hsa-miR-4465, hsa-miR-497-5p, hsa-miR-582-5p, hsa-miR-6083, hsa-miR-708-5p, hsa-miR-9-5p, hsa-miR-92a-3p, hsa-miR-96-5p and hsa-miR-98-3p.

The miRNAs targeting PQBP1 expressed in the exosomes purified from the culture supernatant of dental pulp-derived stem cells were the PQBP1 suppression-related miRNA group (two kinds) below.
hsa-miR-6727-3p and hsa-miR-6727-5p.

There were 153 miRNAs which were expressed in the exosomes of dental pulp-derived stem cells and which were expected to treat Alzheimer's disease, and the results of the comparison of the expression levels thereof are shown in the heat map of FIG. 13. The shades of the heat map indicate the log ratios of the read count values.

In Test Example 3 above, microRNAs which suppress β secretase (BACE1) or APP, which are proteins involved in Alzheimer's disease, and microRNAs which suppress NMDA, inhibit GSK-3β or suppress PQBP1, which are target proteins of a therapeutic agent, were found.

In particular, from FIG. 13 and FIG. 13-2, it was found that the 859 miRNAs which were observed to be expressed in the cerebral cortex and which were observed also in the gene expression suppressor of Example 1 included 153 miRNAs having the effect of suppressing amyloid β- or tau protein-related genes (responsible genes for Alzheimer's disease or the like) as targets.

### [Test Example 4]: Gene Expression Regulation

To cerebral cortex neurons (neurons derived from an Alzheimer's disease patient), the exosomes of dental pulp-derived stem cells obtained in Example 1 or the exosomes of adipose-derived stem cells of Comparative Example 1 were added. The added amount was 1,000 exosomes per cerebral cortex neuron. After 72 hours, the cerebral cortex neurons were recovered, and the mRNAs were purified. Regarding the purified mRNAs, the expression of the genes as the subjects of expression analysis related to Alzheimer-type dementia (with elevated expression) were quantified by qPCR.

On the other hand, cerebral cortex neurons without the addition of the exosomes were used as the control (Reference Example 1). The mRNAs of the recovered cerebral cortex neurons were purified, and the expression of the genes as the subjects of expression analysis were quantified by the same method as that in Example 1 and Comparative Example 1.

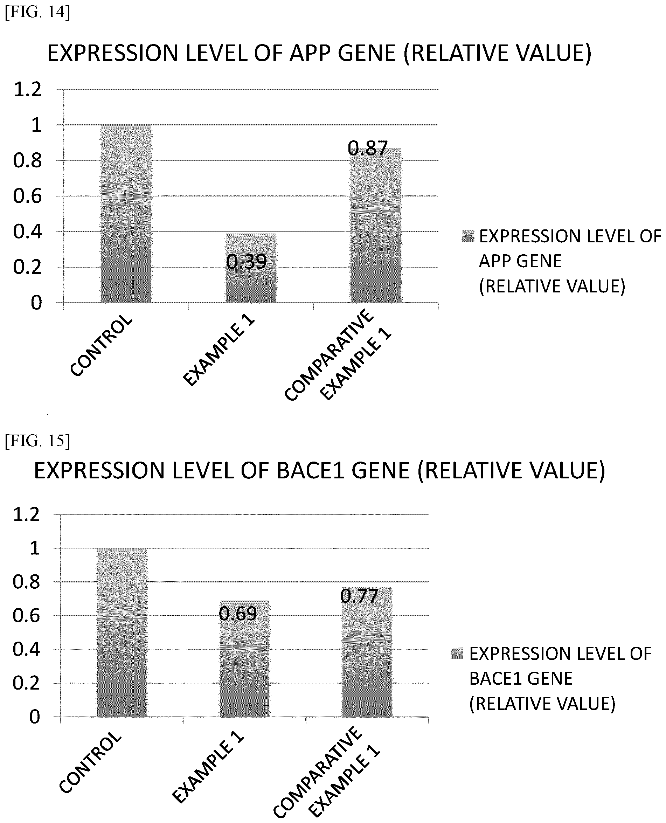

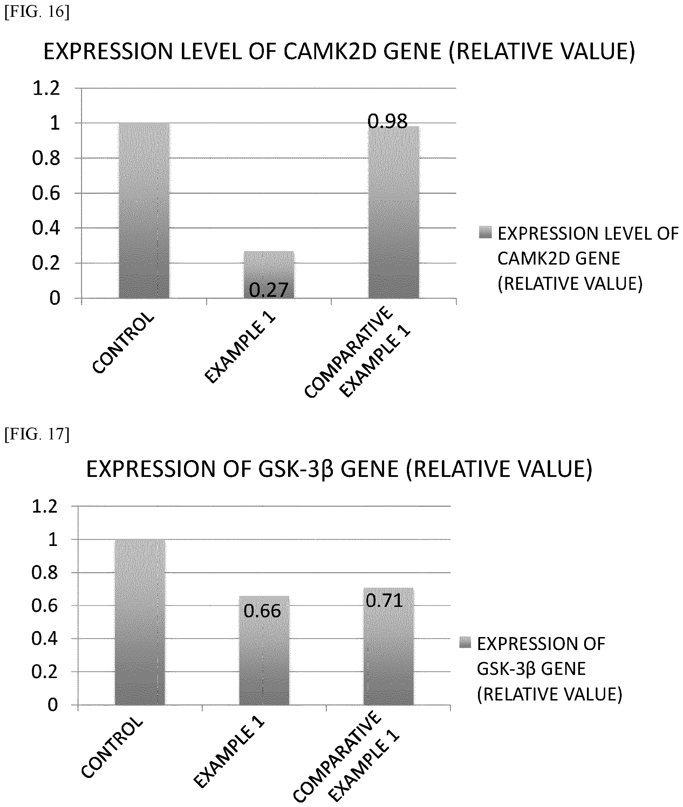

The expression levels of the genes as the subjects of expression analysis of Example 1 (cells treated with the exosomes of dental pulp-derived stem cells) and Comparative Example 1 (cells treated with the exosomes of adipose-derived stem cells) were calculated as the relative values where the expression levels of the genes as the subjects of expression analysis of Reference Example 1 (control; untreated cells) were regarded as 1.0. The genes as the subjects of expression analysis were five kinds, namely amyloid precursor protein (APP) gene, β-secretase (BACE1) gene, an NMDA-activating protein (CAMK2D) gene, glycogen synthase kinase-3β (GSK-3β) gene and polyglutamine-binding protein-1 (PQBP1) gene. The obtained results of the expression levels of the genes as the subjects of expression analysis are shown in FIGs. 14 to 18.

From the results of expression analysis of the genes shown in FIGs. 14 to 18, it was found that the gene expression regulator of the invention (Example 1) can regulate the expression of the five kinds of genes as the subjects of expression analysis (the genes involved in the expression of APP, BACE1, CAMK2D as an NMDA-activating protein, GSK-3β and PQBP1), which are related to Alzheimer-type dementia (the expression thereof was elevated with the control). In particular, it was found that the expression of the five kinds of genes as the subjects of expression analysis related to Alzheimer-type dementia can be suppressed more significantly by the gene expression regulator of Example 1 compared to the gene expression regulator of Comparative Example 1.

### [Test Example 5]: Kinds and Expression Levels of miRNAs

The miRNAs of the gene expression suppressor of Example 1 (the exosomes of dental pulp-derived stem cells), the gene expression suppressor of Comparative Example 1 (the exosomes derived from adipose tissue stem cells; ADSC) and the gene expression suppressor of Comparative Example 2 (the exosomes derived from umbilical cord stem cells; Placenta-MSC) were analyzed using qPCR in the same manner as in Test Example 4. Of the obtained analysis results, the expression levels of the miRNAs below of a subject gene group of Alzheimer's disease were compared.
hsa-miR-16-5p (APP suppression-related miRNA, BACE1 inhibition-related miRNA and GSK-3β inhibition-related miRNA),
hsa-miR-21-5p (DLG1 suppression-related miRNA),
hsa-let7-a-5p (CAMK2D suppression-related miRNA),
hsa-miR-92a-3p (CAMK2A suppression-related miRNA) and
hsa-miR-22-3p (CAPN1 suppression-related miRNA).

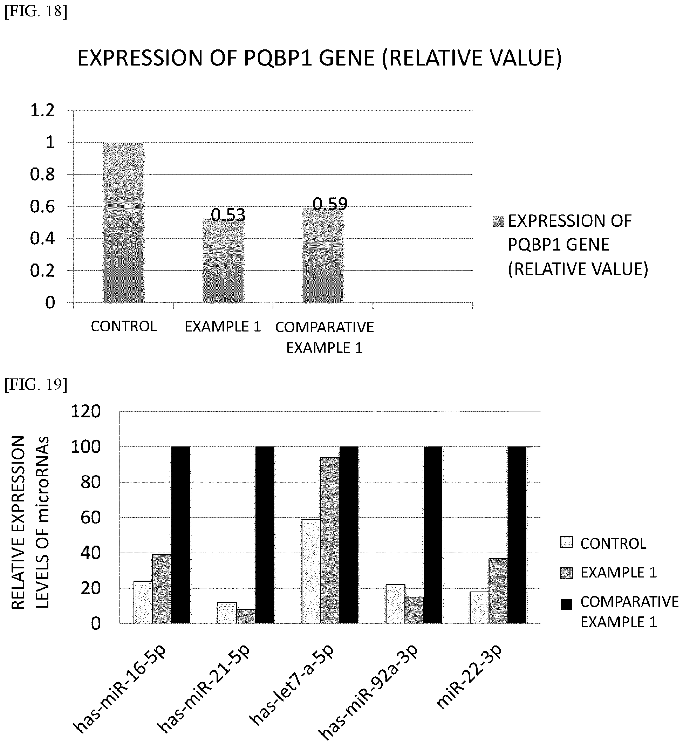

The results are shown in FIG. 19. In FIG. 19, the relative values are shown where the expression levels of the miRNAs in the gene expression suppressor of Example 1 (the exosomes of dental pulp-derived stem cells) are regarded as 100.

From FIG. 19, it was found that the expression levels of the miRNAs that suppress the gene group in which the expression is elevated with relation to Alzheimer's disease (microRNAs which are expected to have a therapeutic effect) were significantly high in the gene expression suppressor of Example 1 (the exosomes of dental pulp-derived stem cells), compared to those in the exosomes derived from other mesenchymal stem cells (the exosomes derived from adipose stem cells of Comparative Example 1 and the exosomes derived from umbilical cord stem cells of Comparative Example 2).

## Claims

1. A gene expression regulator comprising microparticles containing miRNA which targets a gene related to the expression of at least one kind of protein of amyloid precursor protein that is abbreviated as APP, β-secretase that is abbreviated as BACE1, an NMDA-activating protein, glycogen synthase kinase-3β that is abbreviated as GSK-3β, and polyglutamine-binding protein-1 that is abbreviated as PQBP1.

2. The gene expression regulator according to claim 1, wherein the microparticles are exosomes.

3. The gene expression regulator according to claim 1 or 2, wherein the microparticles contain the miRNA which targets the gene related to the expression of at least one kind of protein of the APP, the BACE1, the NMDA-activating protein, the GSK-3β and the PQBP1 at a higher concentration than that of a culture supernatant of dental pulp-derived stem cells.

4. The gene expression regulator according to any one of claims 1 to 3
which is an expression suppressor of a gene related to the expression of the APP,
wherein the microparticles contain miRNA which targets the gene related to the expression of the APP.

5. The gene expression regulator according to claim 4, wherein the microparticles contain at least one kind of the APP suppression-related miRNA group below;
the APP suppression-related miRNA group:
hsa-miR-101-3p, hsa-miR-106b-5p, hsa-miR-1229-5p, hsa-miR-1238-3p, hsa-miR-1260b, hsa-miR-1276, hsa-miR-128-3p, hsa-miR-142-3p, hsa-miR-142-5p, hsa-miR-144-3p, hsa-miR-151a-3p, hsa-miR-153-3p, hsa-miR-15a-5p, hsa-miR-15b-5p, hsa-miR-16-5p, hsa-miR-17-5p, hsa-miR-185-5p, hsa-miR-186-5p, hsa-miR-194-5p, hsa-miR-195-5p, hsa-miR-196a-5p, hsa-miR-203a-3p, hsa-miR-20a-5p, hsa-miR-222-3p, hsa-miR-298, hsa-miR-31-5p, hsa-miR-3120-3p, hsa-miR-3132, hsa-miR-323a-3p, hsa-miR-324-5p, hsa-miR-328-3p, hsa-miR-3620-3p, hsa-miR-3646, hsa-miR-369-3p, hsa-miR-373-3p, hsa-miR-374c-5p, hsa-miR-381-3p, hsa-miR-382-5p, hsa-miR-383-5p, hsa-miR-411-3p, hsa-miR-423-3p, hsa-miR-424-3p, hsa-miR-424-5p, hsa-miR-4484, hsa-miR-455-3p, hsa-miR-4786-5p, hsa-miR-484, hsa-miR-490-5p, hsa-miR-497-5p, hsa-miR-500a-5p, hsa-miR-5093, hsa-miR-532-3p, hsa-miR-532-5p, hsa-miR-539-3p, hsa-miR-548f-3p, hsa-miR-551b-5p, hsa-miR-5584-5p, hsa-miR-567, hsa-miR-5696, hsa-miR-582-5p, hsa-miR-6073, hsa-miR-873-5p and hsa-miR-93-5p.

6. The gene expression regulator according to any one of claims 1 to 5
which is an inhibitor of a gene related to the expression of the BACE1,
wherein the microparticles contain miRNA which targets the gene related to the expression of the BACE1.

7. The gene expression regulator according to claim 6, wherein the microparticles contain at least one kind of the BACE1 inhibition-related miRNA group below;
the BACE1 inhibition-related miRNA group:
hsa-miR-107, hsa-miR-124-3p, hsa-miR-1267, hsa-miR-128-3p, hsa-miR-129-2-3p, hsa-miR-140-3p, hsa-miR-140-5p, hsa-miR-141-3p, hsa-miR-15a-5p, hsa-miR-16-5p, hsa-miR-17-5p, hsa-miR-195-5p, hsa-miR-200a-3p, hsa-miR-203a-3p, hsa-miR-212-3p, hsa-miR-212-5p, hsa-miR-24-3p, hsa-miR-26b-5p, hsa-miR-27a-3p, hsa-miR-298, hsa-miR-299-3p, hsa-miR-29a-3p, hsa-miR-29b-3p, hsa-miR-29c-3p, hsa-miR-328-3p, hsa-miR-339-5p, hsa-miR-340-5p, hsa-miR-369-3p, hsa-miR-374a-5p, hsa-miR-374b-5p, hsa-miR-374c-5p, hsa-miR-382-5p, hsa-miR-421, hsa-miR-424-5p, hsa-miR-455-3p, hsa-miR-497-5p, hsa-miR-505-3p, hsa-miR-532-5p, hsa-miR-7-5p, hsa-miR-874-3p and hsa-miR-9-5p.

8. The gene expression regulator according to any one of claims 1 to 7
which is an inhibitor of a gene related to the expression of the GSK-3β,
wherein the microparticles contain miRNA which targets the gene related to the expression of the GSK-3β.

9. The gene expression regulator according to claim 8, wherein the microparticles contain at least one kind of the GSK-3β inhibition-related miRNA group below;
the GSK-3β inhibition-related miRNA group:
hsa-let-7a-3p, hsa-let-7b-3p, hsa-let-7f-1-3p, hsa-let-7f-2-3p, hsa-miR-101-3p, hsa-miR-1185-1-3p, hsa-miR-1185-2-3p, hsa-miR-124-3p, hsa-miR-128-3p, hsa-miR-129-5p, hsa-miR-132-3p, hsa-miR-137, hsa-miR-140-5p, hsa-miR-142-5p, hsa-miR-144-3p, hsa-miR-150-5p, hsa-miR-155-5p, hsa-miR-15a-5p, hsa-miR-15b-5p, hsa-miR-16-5p, hsa-miR-1910-5p, hsa-miR-195-5p, hsa-miR-199a-5p, hsa-miR-212-3p, hsa-miR-218-5p, hsa-miR-219a-5p, hsa-miR-23a-3p, hsa-miR-24-3p, hsa-miR-26a-5p, hsa-miR-26b-5p, hsa-miR-27a-3p, hsa-miR-28-5p, hsa-miR-29a-3p, hsa-miR-29b-3p, hsa-miR-346, hsa-miR-369-3p, hsa-miR-374a-5p, hsa-miR-374b-5p, hsa-miR-374c-5p, hsa-miR-377-3p, hsa-miR-409-3p, hsa-miR-409-5p, hsa-miR-410-3p, hsa-miR-424-5p, hsa-miR-425-5p, hsa-miR-4465, hsa-miR-497-5p, hsa-miR-582-5p, hsa-miR-6083, hsa-miR-708-5p, hsa-miR-9-5p, hsa-miR-92a-3p, hsa-miR-96-5p and hsa-miR-98-3p.

10. The gene expression regulator according to any one of claims 1 to 9, wherein the microparticles are of at least one kind of an expression suppressor of a gene related to the expression of the APP, an inhibitor of a gene related to the expression of the BACE1 and an inhibitor of a gene related to the expression of the GSK-3β and contain hsa-miR-16-5p.

11. The gene expression regulator according to any one of claims 1 to 10
which is an expression suppressor of the NMDA-activating protein,
wherein the NMDA-activating protein is at least one kind of protein of DLG1, CAMK2D, CAMK2A and CAPN1, and
the microparticles contain a miRNA group which targets a gene related to the expression of at least one kind of protein of DLG1, CAMK2D, CAMK2A and CAPN1, and the microparticles contain at least one kind of miRNA group among the DLG1 suppression-related miRNA group below, the CAMK2D suppression-related miRNA group below, the CAMK2A suppression-related miRNA group below and the CAPN1 suppression-related miRNA group below;
the DLG1 suppression-related miRNA group:
hsa-miR-1-3p, hsa-miR-142-5p, hsa-miR-204-5p, hsa-miR-206, hsa-miR-21-5p, hsa-miR-218-5p, hsa-miR-340-5p and hsa-miR-613,
the CAMK2D suppression-related miRNA group:
hsa-let-7a-5p, hsa-miR-101-3p, hsa-miR-129-5p, hsa-miR-139-5p, hsa-miR-144-3p, hsa-miR-145-5p, hsa-miR-185-5p, hsa-miR-203a-3p, hsa-miR-204-5p, hsa-miR-211-5p, hsa-miR-24-3p, hsa-miR-27a-3p, hsa-miR-30a-3p, hsa-miR-31-5p, hsa-miR-361-5p, hsa-miR-421, hsa-miR-484, hsa-miR-494-3p, hsa-miR-505-3p and hsa-miR-7-5p,
the CAMK2A suppression-related miRNA group:
hsa-miR-129-5p, hsa-miR-137, hsa-miR-142-5p, hsa-miR-148a-3p, hsa-miR-149-3p, hsa-miR-152-3p, hsa-miR-25-3p, hsa-miR-27a-3p, hsa-miR-32-5p, hsa-miR-338-3p, hsa-miR-340-5p, hsa-miR-363-3p, hsa-miR-3665, hsa-miR-4534, hsa-miR-4665-5p, hsa-miR-4688, hsa-miR-485-5p, hsa-miR-5010-5p, hsa-miR-505-5p, hsa-miR-5698, hsa-miR-625-5p and hsa-miR-92a-3p, and
the CAPN1 suppression-related miRNA group:
hsa-miR-1-3p, hsa-miR-124-3p, hsa-miR-140-5p, hsa-miR-17-3p, hsa-miR-22-3p, hsa-miR-34a-5p and hsa-miR-6511b-5p.

12. The gene expression regulator according to claim 11,
wherein the microparticles contain all of
hsa-miR-21-5p of the DLG1 suppression-related miRNA group,
hsa-let-7a-5p of the CAMK2D suppression-related miRNA group,
hsa-miR-92a-3p of the CAMK2A suppression-related miRNA group and
hsa-miR-22-3p of the CAPN1 suppression-related miRNA group.

13. The gene expression regulator according to any one of claims 1 to 12
which is an expression suppressor of the PQBP1,
wherein the microparticles contain miRNA which targets a gene related to the expression of the PQBP1.

14. The gene expression regulator according to claim 13, wherein the microparticles contain at least one kind of the PQBP1 suppression-related miRNA group below;
the PQBP1 suppression-related miRNA group: hsa-miR-6727-3p and hsa-miR-6727-5p.

15. The gene expression regulator according to any one of claims 1 to 14,
wherein the microparticles are microparticles purified and isolated from a culture supernatant of dental pulp-derived stem cells, and
the gene expression regulator does not contain any component obtained by removing exosomes from the culture supernatant of the dental pulp-derived stem cells.

16. An agent for preventing or treating Alzheimer's disease comprising the gene expression regulator according to any one of claims 1 to 15.

17. A method for improving dementia comprising administering an effective amount of the gene expression regulator according to any one of claims 1 to 15 or an effective amount of the agent for preventing or treating Alzheimer's disease according to claim 16 to a subject which has developed amyloid β-related or tau protein-related dementia.
